Europäisches Patentamt

European Patent Office

Office européen des brevets

(19)

(11) EP 0 777 854 B1

(12) EUROPÄISCHE PATENTSCHRIFT

(45) Veröffentlichungstag und Bekanntmachung des Hinweises auf die Patenterteilung:
16.12.1998 Patentblatt 1998/51

(21) Anmeldenummer: 95931186.1

(22) Anmeldetag: 22.08.1995

(51) Int Cl.⁶: G01N 21/27, A61B 5/00

(86) Internationale Anmeldenummer:
PCT/EP95/03337

(87) Internationale Veröffentlichungsnummer:
WO 96/06343 (29.02.1996 Gazette 1996/10)

(54) **VERFAHREN ZUR VALIDIERUNG UND/ODER KALIBRATION VON VORRICHTUNGEN ZUR FOTOMETRIE LEBENDER GEWEBE SOWIE VORRICHTUNG ZUR DURCHFÜHRUNG DES VERFAHRENS**

METHOD OF VALIDATING AND/OR CALIBRATING DEVICES USED FOR CARRYING OUT PHOTOMETRY OF LIVING TISSUES AND A DEVICE FOR IMPLEMENTING SAID METHOD

PROCEDE DE VALIDATION ET/OU D'ETALONNAGE DE DISPOSITIFS SERVANT A LA PHOTOMETRIE DE TISSUS VIVANTS ET DISPOSITIF PERMETTANT D'APPLIQUER LEDIT PROCEDE

(84) Benannte Vertragsstaaten:
**DE FR GB**

(30) Priorität: **22.08.1994 DE 4429758**

(43) Veröffentlichungstag der Anmeldung:
**11.06.1997 Patentblatt 1997/24**

(73) Patentinhaber: **BUSCHMANN, Johannes**
**D-81543 München (DE)**

(72) Erfinder:
• **BUSCHMANN, Johannes**
**D-81543 München (DE)**

• **FALKOWSKI, Reinhold**
**D-80798 München (DE)**

(74) Vertreter: **Alber, Norbert et al**
**Patent- und Rechtsanwälte**
**Hansmann, Vogeser, Dr. Boecker,**
**Alber, Dr. Strych, Liedl**
**Albert-Rosshaupter-Strasse 65**
**81369 München (DE)**

(56) Entgegenhaltungen:
**EP-A- 0 314 331          CH-A- 682 627**
**US-A- 4 834 532          US-A- 5 111 817**
**US-A- 5 278 627**

## Beschreibung

Die Erfindung betrifft die Kalibration und/oder Validierung von Pulsoximetern bzw. Pulsoximetrie-Sensoren, insbesondere für die Anwendung am ungeborenen Kind (Fetus).

Pulsoximetrie ist ein Verfahren zur Messung der arteriellen Sauerstoff-Sättigung. Es läßt sich prinzipiell bei allen pulsatil (systolischer - diastolischer Blutdruck) durchbluteten Geweben aller Spezies einsetzen.

Die Pulsoximetrie wird seit vielen Jahren mit großem Erfolg klinisch eingesetzt. Sie ist aus Operationsräumen, Intensivstationen, Notarztwägen, Neugeborenenstationen usw. nicht mehr wegzudenken. Ihr Erfolg beruht auf der einfachen Handhabung, der zuverlässigen Aussage und der problemlosen Interpretation der angezeigten Werte.

Für die Geburtshilfe ist die Pulsoximetrie bis zum heutigen Tage nicht verfügbar. Dies ist bemerkenswert, denn die Sauerstoff-Sättigung wäre eine sehr wichtige Information für den Geburtshelfer,

- erstens um Sauerstoffmangelzustände frühestmöglich diagnostizieren zu können und
- zweitens um die viel zu häufig gestellte Kaiserschnitt-Indikation (16% Deutschland, 20% Europa, 25-33% USA) präzisieren zu können.

Es ist also naheliegend, daß mehrere Arbeitsgruppen daran arbeiten, dieses Verfahren für die Geburtshilfe zu erschließen. Ein Autor dieser Patentanmeldung hält selbst ein grundlegendes Patent für die Transmissionspulsoximetrie am ungeborenen Feten während der Geburt.

Bei der Entwicklung der Pulsoximetrie für die Geburtshilfe ergibt sich eine besondere Schwierigkeit: Die Kalibration. Darunter versteht man die Herstellung eines Zusammenhanges zwischen der vom Pulsoximeter angezeigten fetalen Sauerstoff-Sättigung und der wirklich im Blut des Feten vorliegenden arteriellen Sauerstoff-Sättigung. Die Kalibration erweist sich auf fetalem Gebiet deshalb als besonders schwierig, weil der Fetus schon physiologischerweise eine sehr niedrige Sauerstoff-Sättigung hat bzw. haben kann. Man muß also für die Kalibration unter anderem sehr niedrige arterielle Sauerstoff-Sättigungen erzeugen, die normalerweise mit dem Leben (sei es Mensch oder Tier) nicht vereinbar sind, zumindest nicht in allen Situationen, in denen das Gehirn mit in die niedrigen arteriellen Sauerstoff-Sättigungen einbezogen ist. Dies trifft nicht zu, wenn ein Körperteil, z.B. ein Arm, für die KalibrationNalidierung vorübergehend isoliert, d.h. anders durchblutet wird als der Rest des Körpers in Durchblutungseinheit mit dem Gehirn.

Der Fetus ist für diese physiologische Sauerstoff-Mangel-Situation mit einer speziellen Hämoglobinvariante ausgestattet, dem fetalen Hämoglobin (HbF). Es weist eine linksverschobene Sauerstoffbindungskurve auf, hat also eine besonders hohe Affinität zu Sauerstoff und ermöglicht damit selbst unter physiologisch ungünstigen Verhältnissen (längere sauerstoffarme Phasen [Wehen, Austreibungsphase], erschwerte Diffusion [Plazenta], Mischung von arteriellem mit venösem Blut [physiologische Shunts]) noch Sauerstoff zu binden und den fetalen Geweben schließlich zur Verfügung zu stellen.

Da alle auf dem Markt befindlichen Pulsoximeter vom Hersteller kalibriert werden mußten, wurden einige Methoden erarbeitet und in der Literatur beschrieben. Die Kalibration über einen großen Sauerstoff-Sättigungsbereich, wie sie für die fetale Situation nötig ist, stellt jedoch eine besondere Anforderung an den Hersteller entsprechender Geräte dar, die mit den bekannten Methoden nicht bewältigt werden können.

Der Stand der Technik beschreibt einige, letztlich naheliegende Kalibrationsmethoden:

1. Kalibration an freiwilligen Probanden: Hier atmen junge, gesunde Probanden ein Gemisch aus Sauerstoff und Stickstoff (und Kohlendioxid), dessen Sauerstoffpartialdruck in definierten Schritten von normalen Werten auf hypoxische Werte herabgesenkt wird. Nachdem sich ein Gleichgewicht zwischen dem Sauerstoff-Partialdruck im Gasgemisch und im Blut eingestellt hat werden Blutproben genommen, aus denen die Sauerstoff-Sättigung im arteriellen Blut bestimmt wird und gleichzeitig, d.h. im Moment der Blutentnahme wird die Meßgröße Omega (siehe unten) mit dem zu kalibrierenden Pulsoximeter gemessen. Die Kalibration besteht nun darin, möglichst viele und solide Meßpunkte zu erhalten, für die sowohl die Sauerstoff-Sättigung als auch der Wert Omega bekannt sind.

In das Gerät, d.h. in die Software des Gerätes wird dann eine Tabelle oder Kurve oder Formel eingegeben, die es ermöglicht, daß zu jedem gemessenen Omega eine korrespondierende Sauerstoff-Sättigung ermittelt werden kann (Severinghaus / San Francisco: Pulse Oximetry, Springer-Verlag, 1986).

2. Eine andere Methode besteht darin, die Pulsationen arteriellen Blutes anstatt in einem lebenden Gewebe in einem künstlichen Aufbau zu fotometrieren. Dazu wird z.B. ein künstlicher Finger aus einem (roten) Gießharz gefertigt. Ein Loch wird in das transparente Material gebohrt, in das eine Achse eingebracht wird, in die am Ende ein Schlitz eingesägt wurde. In diesen Schlitz wird eine rote Filterscheibe eingeklemmt. Wird dieser Testfinger in einen Pulsoximeter-Fingersensor eingebracht und wird weiter die Achse gedreht, so entstehen durch die winkelabhängige Veränderung der Farbstoff-Schichtdicke Transmissionsschwankungen, die ein Gewebe imitieren können. Es wird beschrieben, daß je nach Farbstoffkonzentration in der Filterscheibe Sauerstoff-Sättigungswerte von 50 bis 100% imitiert werden können (A.J.Munley, A. Shaw, M.J.Sik in The Lancet, Mai 13, 1989). Anders als bei einem wirklichen Finger kommen keine Änderungen des mittleren effektiven Lichtweges zwischen Lichtsendern und Lichtempfänger zustande, der Fingersensor bleibt unbewegt. Das System ist sehr artifiziell. Offen bleibt zudem wie der Testfinger seinerseits kalibriert wird.

Da die physiologische und/oder pathologische Sauerstoff-Sättigung beim ungeborenen Feten unter der Geburt weniger als 10% betragen kann, und solch niedrige Sauerstoff-Sättigungswerte nach der Geburt mit dem Leben nicht vereinbar sind, bedarf es eines völlig neuen Kalibrations-Konzeptes. Eine wesentliche Grundlage dieses Kalibrations-Konzeptes muß es sein, ein Gewebe bzw. Gewebemodell zu finden, das arterielle Sauerstoff-Sättigungen im gesamten Bereich von 100% bis hinunter zu (beinahe) 0% zuläßt, ohne daß der Sauerstoffmangel die Validität des Modells gefährdet.

Ausgehend von dem oben beschriebenen Stand der Technik ist es daher Aufgabe der vorliegenden Erfindung, ein Verfahren zur Validierung von Vorrichtungen zur Fotometrie lebender Gewebe zur Verfügung zu stellen, welches die Erfassung (Konzentration) einer in einer Körperflüssigkeit zu bestimmenden Substanz über einen möglichst weiten Konzentrationsbereich ermöglicht. Die Erfindung löst diese Aufgabe mit einem Verfahren wie es in Anspruch 1 angegeben ist. Die Unteransprüche stellen bevorzugte Ausführungsformen der vorliegenden Erfindung dar.

Validierung bedeutet im Rahmen der vorliegenden Erfindung, daß die Gültigkeit (Richtigkeit) angezeigter Werte (Meßwerte) überprüft wird; beispielsweise ist damit auch die Gültigkeit der Kalibrierung eines Pulsoximeters einbezogen.

Bei der erfindungsgemäßen Kalibrationsmethode weist die Fotometrie der Körperflüssigkeit ganz andere geometrisch-optische Verhältnisse auf als im Stand der Technik. Der Stand der Technik setzt zur Kalibration als Küvetten dienende Hohlkörper ein, die es erlauben, die Schichtdicke rhythmisch zu verändern, um das optisch-plethysmografische Signal zu simulieren. So werden im Stand der Technik Anordnungen beschrieben, daß Absorptionsschwankungen zur Imitation von Gewebe dadurch zustande kommen, daß Blut oder eine andere geeignet gefärbte Flüssigkeit im Inneren eines Schlauches Druckschwankungen unterworfen werden, so daß dieser Schlauch Dickenschwankungen ausführt. Diese durch den Druck der Flüssigkeit selbst ausgelösten Dickenschwankungen entsprechen Absorptionsschwankungen, die eine Simulation von Gewebe bewirken sollen. Dabei sind Lichtsender, Küvette und Lichtempfänger so angeordnet, als handele es sich um die Fotometrie nicht-streuender Flüssigkeiten, bei denen es völlig ausreicht, wenn die Farbstofflösung irgendwie im geraden Strahlengang zwischen Lichtsender und -empfänger angeordnet ist.

Da aber bei Blut und vor allem bei (durchblutetem) Gewebe stark streuende Verhältnisse vorliegen, geben die Anordnungen entsprechend dem Stand der Technik die Verhältnisse im Gewebe nicht wieder, insbesondere nicht die Verhältnisse einer invasiven fetalen Pulsoximetrie. Um diese optischen Verhältnisse bestmöglichst zu simulieren, muß berücksichtigt werden,

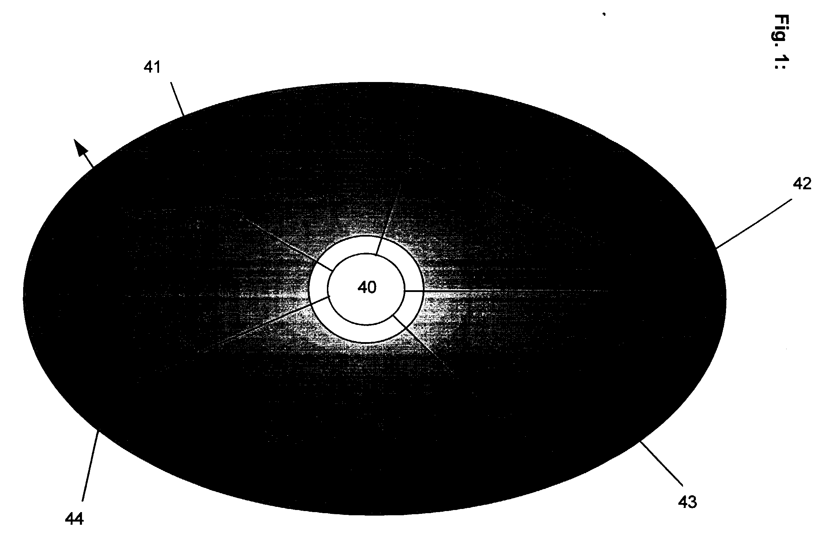

1. daß sich das Licht vom Emitter aus in alle Raumrichtungen ausbreitet und
2. daß der Farbstoff, Blut, bzw. durchblutetes Gewebe in allen Raumrichtungen vorhanden ist, also absorbiert und streut und
3. daß nicht eine geometrische Begrenzung, z.B. durch eine Küvette gegeben ist, sondern die Begrenzung des dreidimensional durchstrahlten Gewebes durch Intensitätsabfall bedingt ist, d.h. bei entsprechend langen Lichtwegen, auf denen das Licht durch Streuung und Absorption geschwächt wird, verringert sich die Lichtintensität so, daß schließlich der Beitrag zum Gesamtsignal irrelevant wird (man könnte von einer "intensitätsbegrenzten Küvette" sprechen, Fig. 1).

Die Simulation dieser optischen Verhältnisse gelingt erfindungsgemäß am besten durch eine Anordnung, bei der Lichtsender und Lichtempfänger entweder im Inneren eines mehr oder weniger vollständig mit Blut gefüllten Hohlkörpers angeordnet sind (Fig. 1), oder innerhalb je eines spektral neutralen Streukörpers, so, daß das Licht auf dem Weg vom Sender-Streukörper zum Empfänger-Streukörper durch Blut möglichst homogen, d.h. unter Vermeidung jeder Inhomogenität der Strahlungsdichte [= optische Shunts]) verläuft und keine optischen Abkürzungen (Shunts) möglich sind. Durch die Verwendung von Streukörpern wird

1. das Streuniveau von Gewebe erreicht, das deutlich höher ist als das vom Blut allein und
2. eine weitgehende Unabhängigkeit von der Streuwirkung des Blutes erreicht, die mit dem Hämoglobingehalt, genauer mit der Anzahl der Zellen, stark schwanken kann.

Die erfindungsgemäße Kalibrationsmethode ist überhaupt die konsequente Simulation lebenden Gewebes insbesondere für den Einsatz invasiver Pulsoximetrie-Sensoren.

Für ein diffus streuendes Medium wie Blut ist bei den erfindungsgemäßen Kalibrationsanordnungen ein meßbarer, direkter Lichtweg zwischen Lichtemitter und -empfänger entsprechend dem Lambert-Beer'schen Gesetz eher die Ausnahme (sehr geringe Hämoglobingehalte, die eine relativ gering Streuung bewirken, seien hier vernachlässigt)(siehe Figur 1). Durch Streuung vor allem durch Gewebes bedingt und Absorption vor allem durch Blut hervorgerufen nimmt die Lichtintensität vom Lichtsender her in allen Richtungen stark ab. Nur ein Teil des mit Blut gefüllten Raumes (= physikalischen Küvette) enthält also (für Signal und/oder Störsignale) relevante Lichtintensitäten (intensitätsbegrenzte Küvette).

Bei durch Streuung vielfach abgewinkelten Lichtwegen werden die realen Verhältnisse korrekter durch statistische Lichtverläufe beschrieben: der Lichtweg zwischen Lichtemitter und -empfänger hat nun eine bestimmte **Verteilung** und damit eine **mittlere Lichtweglänge.** Entsprechend ergibt sich auch eine mittlere Absorption usw. Die direkte Zuwendung von Lichtemitter und -empfänger zueinander wird irrelevant.

Kommen wir nun zur Dynamik der Lichtwege, ein wichtiges Element für die Kalibration der Pulsoximetrie. Eine geeignete Vorrichtung muß ja eine Veränderung des Lichtweges erzeugen, erfindungsgemäß handelt es sich um eine Veränderung des **mittleren Lichtweges.** Der effektive Abstand zwischen Sender und Empfänger der oben erwähnten Lichtschranke wird nun rhythmisch verändert entsprechend dem Prinzip der Pulsoximetrie. Dies läßt sich erfindungsgemäß bewerkstelligen z.B. durch eine echte Veränderung der Position des Senders oder Empfängers, oder durch Eindringen einer optischen Diskontinuität bzw. eines optischen Trennkörpers, z.B. eines diffusen, farbneutralen Keils in die Blutschicht zwischen Emitter und Empfänger, der die "mittlere effektive Lichtweglänge" bzw. die effektive Absorptionslänge "durch die Körperflüssigkeit aktiv auf definierte Art dynamisch verändert, ohne die Körperflüssigkeit zur Übertragung von Kräften einzusetzen".

Gegenstand der Erfindung sind insbesondere Vorrichtungen zur Durchführung des erfindungsgemäßigen Verfahrens mit:

- wenigstens einer Einrichtung zum Einstellen der Konzentration einer in einer Körperflüssigkeit zu erfassenden Substanz;
- wenigstens einer Transporteinrichtung, womit die auf eine bestimmte Konzentration eingestellte Körperflüssigkeit zu wenigstens einer Meßzelle gebracht wird;
- wenigstens einem Sender und wenigstens einem Empfänger, welche in der Meßzelle angeordnet sind, so daß der mittlere effektive Lichtweg zueinander dynamisch auf definierte Art veränderbar ist, ohne daß zur Änderung des mittleren effektiven Lichtweges die Körperflüssigkeit zur Übertragung von Kräften auf Sender und/oder Empfänger eingesetzt wird; und
- einer Einrichtung, welche eine mittels des Empfängers erfaßte Lichtintensität wenigstens eines spektralen Fensters, welche die Körperflüssigkeit durchdrungen hat, in Form wenigstens eines geeigneten Parameters ausgibt und in einen Zusammenhang zwischen der Konzentration einerseits und dem Parameter andererseits bringt.

Alternativ weisen diese Vorrichtungen auf:

- wenigstens einen Sender und wenigstens einen Empfänger, welche in der Meßzelle angeordnet sind, wobei in einem optischen Raum zwischen Sender und Empfänger eine optische Diskontinuität mit veränderbarer Dicke vorgesehen ist.

Diese Vorrichtungen zur Validierung dienen im allgemeinen zur Kalibration, insbesondere zur in vitro-Kalibration. Es handelt sich bei diesen Vorrichtungen regelmäßig um Geräte zur Pulsoximetrie. Die in diesen Vorrichtungen eingesetzt Körperflüssigkeit ist vorzugsweise Blut, insbesondere menschliches Blut. Die Konzentration einer in einer Körperflüssigkeit zu erfassenden Substanz ist regelmäßig die Sauerstoff-Sättigung. In der erfindungsgemäßen Vorrichtung sendet der Sender Licht zweier spektraler Fenster, bevorzugt Rot- und Infrarotlicht, insbesondere für die Pulsoximetrie durch die Körperflüssigkeit, dessen Intensität nach Durchdringen der Körperflüssigkeit mittels eines Fotosensors erfaßt wird. Ganz besonders bevorzugt wird für den roten Bereich eine Wellenlänge zwischen etwa 560 und 680 nm und für den infraroten Bereich 760-1040 nm. In der Vorrichtung wird der mittlere effektive Lichtweg zwischen Sender und Empfänger durch magnetische und/oder mechanische Kräfte variiert wird. Die optische Diskontinuität kann in Form eines - bezogen auf das spektrale Fenster - optisch durchlässigen Körpers, beispielsweise aus Glas, mit sich wiederholender veränderlicher Dicke vorliegen. Dieser Körper kann eine rotierende optisch durchlässige Scheibe mit unterschiedlicher Dicke oder ein rhythmisch bewegter Keil sein. Vorzugsweise ist der Sender und/oder Empfänger zur Verringerung der nicht-pulsatilen Absorptionslänge in der Körperflüssigkeit, insbesondere im Blut beschichtet ist, wobei die bevorzugte Beschichtung ein Glas oder einen Kunststoff, insbesondere Harz, vorzugsweise Epoxyharz ist .Die Beschichtung kann zusätzlich Teilchen enthalten, die in die Schicht auf Sender und/oder dem Empfänger eindispergiert sind, wobei Glasperlen, insbesondere solche von ca. 1 μm Durchmesser, oder Titandioxid bevorzugt werden.

Vorzugsweise werden in die Körperflüssigkeit, insbesondere Blut, zur besseren Gewebeimitation Streuteilchen einsuspendiert, insbesondere solche aus Kunststoff wie PE (Polyethylen), HDPE (High Density Polyethylen) oder dergleichen, welche in etwa die spezifische Dichte der Körperflüssigkeit aufweisen, und welche einen solchen Durchmesser aufweisen, daß sie einen Faseroxigenator passieren können.

Anders ausgedrückt betrifft die Erfindung auch Verfahren zur optischen Simulation von menschlichem Gewebe für die Pulsoximetrie, welches die folgenden Schritte umfaßt:

- In vitro-Einstellen einer bestimmten Sauerstoffkonzentration im Blut;
- Transportieren des auf die bestimmte Sauerstoffkonzentration eingestellten Blutes zu wenigstens einer Meßzelle;
- Erfassen einer Lichtintensität in wenigstens einem spektralen Fenster zur Bestimmung mindestens eines geeigneten Parameters in dem Blut, welches in der Meßzelle enthalten ist, mittels wenigstens einem Sender und wenigstens einem Empfänger,

wobei man die effektive Absorptionslänge durch die Körperflüssigkeit aktiv auf definierte Art dynamisch verändert, ohne die Körperflüssigkeit zur Übertragung von Kräften einzusetzen, wobei die Körperflüssigkeit den optischen Raum zwischen Sender und Empfänger einnimmt.

Ganz besonders vorteilhaft sind Pulsoximeter, die mittels des erfindungsgemäßen Verfahrens kalibriert wurden. Es mag zwar im Stand der Technik entsprechende Pulsoximeter geben, die äußerlich von erfindungsgemäßen nicht zu unterscheiden sind. Die Genauigkeit einer Anzeige läßt sich aber über einen Vergleich mehrerer Pulsoximeter quantitativ erfassen. Ein weniger akkurat nach dem Stand der Technik kalibriertes Pulsoximeter mag zufallsbedingt einmal ein der Realität entsprechendes Resultat liefern. Für die erfindungsgemäßen Pulsoximeter läßt sich aber wiederholbar diese Genauigkeit bei der Anzeige einstellen, wie sie im Stand der Technik nicht zu erreichen ist. Es kann also immer im Vergleich ermittelt werden, daß bei erfindungsgemäß kalibrierten Pulsoximetern das Verfahren die Pulsoximeter mit einer neuen Eigenschaft ausstattet.

Bei der Erfindung werden die zu kalibrierenden Sensoren in Blut mit einer in beliebigen Grenzen (0-100%) einstellbaren Sauerstoff-Sättigung (Tonometer, [Faser-]Oxigenator mit Gemischen aus $N_2$, $O_2$ und eventuell $CO_2$) und bekannten (CO-Oximeter) so eingebracht, daß der effektive Lichtweg (Streuung eingeschlossen) zwischen den Lichtsendern und dem Lichtempfänger vollständig in Blut verläuft. Es wird also eine Fotometrie durchgeführt, bei der sich zwischen Lichtsender und - Empfänger Blut einer Schichtdicke befindet, die nicht durch Küvettenmaße definiert ist, sondern dadurch, daß die Streulichtwege desto länger werden, je stärker sie sich vom direkten Weg zwischen Sender und Empfänger entfernen, bis die Lichtintensität schließlich so klein wird, daß der Beitrag zum Signal irrelevant wird. Man könnte von einer virtuellen oder funktionellen Küvette sprechen. Es existiert also eine Verteilung des effektiven Lichtweges mit einer mittleren Länge. Wird die Länge des effektiven Lichtwegs in Blut repetitiv (z.B. periodisch) verändert, sei es durch Veränderung des mittleren effektiven Lichtweges zwischen Sender und Empfänger, sei es durch Beeinflussung der Absorption und/oder Streuung im effektiven Lichtweg, so entsteht ein typisches Signal mit einem konstanten und einem pulsatilen Anteil, so daß aus den Minima und Maxima des Gesamtsignals die Zwischengröße $\Omega$ (Formel siehe unten) errechnet werden kann. Konkret kann man die Absorption ändern indem z.B. ein optisch durchlässiger Körper (z.B. in Keilform) z.B. dynamisch in den Strahlengang eingebracht wird, der die absorbierende Flüssigkeit (Blut) zumindest teilweise verdrängt und somit die wirksame Schichtdicke verändert. Alternativ kann ein Körper statisch eingebracht werden, dessen optische Oberflächeneigenschaften, z.B. die Reflexivität, sich ändern. Auch damit läßt sich der mittlere Lichtweg und damit die effektive Absorptionsschichtdicke dynamisch ändern

Die gewünschte Kalibrationskurve ergibt sich aus dem Zusammenhang der im Blut eingestellten Sauerstoff-Sättigungen mit den aus den gemessenen Lichtintensitäten berechneten Werten für $\Omega$. Dieses Verfahren bzw. diese Vorrichtung läßt sich nicht nur für die verschiedensten Arten von Pulsoximetriesensoren (Reflex-, Transmissionssensoren) anwenden, sondern allgemein für alle Anwendungen, bei denen Konzentrationen bestimmter Substanzen in Flüssigkeiten und besonders Körperflüssigkeiten z.B. durch die Fotometrie lebenden Gewebes bestimmt werden sollen.

$$\Omega = \frac{\ln\left(\dfrac{I_{out\,max\,\lambda1}}{I_{out\,min\,\lambda1}}\right)}{\ln\left(\dfrac{I_{out\,max\,\lambda2}}{I_{out\,min\,\lambda2}}\right)}$$

Erfindungsgemäß wurde von folgenden Überlegungen ausgegangen:

Humanexperimentell: Es gibt drei Möglichkeiten, direkt menschliches Gewebe für die KalibrationΝalidierung über einen weiten Sauerstoff-Sättigungsbereich einzusetzen und doch zu gewährleisten, daß die Gefahr einer Hirnschädigung sicher ausgeschlossen ist:

- bestehender Hirntod
- Anenzephalie

- vom gesunden Hirn abgekoppeltes Gewebe mit eigenem Kreislauf, z.B. von Herz-Lungen-Maschine perfundierte Extremität (Arm oder Bein)
  [Herz-Lungen-Maschine -> Arterie -> Arteriole -> Kapillare -> Venole -> Vene -> Herz-Lungen-Maschine]

Ein völlig anderer Ansatz, Absorptionsänderungen durch Perfusion von Gewebe mit menschlichem Blut zu erhalten ist der tierexperimentelle Ansatz, tierisches Gewebe mit menschlichem Blut zu perfundieren. Die spektralen Eigenschaften menschlichen Blutes sind damit natürlich ideal nachgebildet, allerdings ist nach wie vor das tierische Gewebe in vielen wichtigen Punkten, insbesondere dem histologischen Aufbau abweichend vom menschlichen Blut.

Ein weitere erfindungsgemäße Möglichkeit zur Kalibration, insbesondere invasiver fetaler Pulsoximetriesensoren menschliches Gewebe nutzbar zu machen, besteht darin, die Sauerstoffsättigung einer arteriell und venös geblockten Extremität durch Eigenverbrauch des Gewebes langsam abfallen zu lassen und eine Art Pulsoximetrie zu betreiben, indem man den Sensor einer künstlichen mechanischen Exzitation unterwirft, d.h. z.B. durch Bewegungen des Sensors selbst, die Schichtdicke variiert bzw. Druckschwankungen von außen in das Gewebe fortgeleitet (z.B. oszillierende Manschetten, ähnlich einer Blutdruck-Manschette). Wichtig ist, dabei zu verstehen, daß ein wesentliches Merkmal der Pulsoximetrie verlassen wurde, nämlich das, daß die Transmissionsschwankungen durch Volumenschwankungen der Arteriolen zustande kommen. Bei der "Kapillarbett-Kalibration" wird das gesamte Gewebe Druckschwankungen unterworfen, was eine Verschiebung sämtlicher Gefäßbetten bewirkt: der Arterien und Arteriolen, der Kapillaren sowie der Venolen und Venen. Entsprechend stellt die angezeigte Sauerstoff-Sättigung eine Mischsättigung dar entsprechend der Volumenanteile der verschiedenen Gefäßkomponenten. Dabei ist besonders vorteilhaft, daß die Kapillaren die einzigenGefäße sind, in denen ein nennenswerter Umfang von Sauerstoffaustausch mit dem Gewebe möglich ist. Die Sauerstoffdiffusion ins Gewebe hinein und der Verbrauch des Sauerstoffs durch das Gewebe führt letztlich zu einem kontinuierlichen Absinken der Misch-Sauerstoff-Sättigung. Als Referenz kommen entweder eine venös bzw. arteriell möglichst weit nach distal vorgeschobene faseroptische Sättigungsmeßtechnik in Frage bzw. Misch-Blutproben, möglichst konstanter Mischverhältnisse, z.B. aus einer Fingerbeere, der durchblutungs-geblockten Extremität (des durchblutungs-geblockten Fingers).

Der Stand der Technik (US-Patent 4,883,055) beschreibt lediglich eine fehlerhafte Alternative zur Pulsoximetrie mit artifiziellen Pulsen, wobei das darin beschriebene Verfahren aufgrund eines fehlerhaften Verständnisses der zugrunde liegenden Pulsoximetrie-Prinzipien so nicht funktioniert.

Tierische oder menschliche Gewebe kommen für die extrem niedrigen Sauerstoff-Sättigungen nur dann in Frage, wenn der Sauerstoffmangel keinen Hirntod verursachen kann. Dies ist entweder der Fall, wenn das Hirn schon tot ist (Hirntod oder Anencephalie - wobei jedoch eine Probennahme naturgemäß mit ethischen Problemen verbunden ist), wobei es prinzipiell unerheblich ist, ob der Kreislauf natürlich ist (Herz, Lunge) oder artifiziell (Herz-Lungen-Maschine), oder wenn das Gewebe nicht mit einem gefährdeten Hirn über ein gemeinsames arterielles Gefäßsystem verbunden ist (abgekoppelte Gewebe z.B. isoliert perfundierte Extremität [artifizieller Kreislauf: Arterie -> Arteriole -> Kapillare -> Venole -> Vene -> Herz-Lungen-Maschine] -> Arterie). Dabei ist zu beachten, daß sich hypoxische Gewebe anders verhalten können, insbesondere der arterielle Gefäßtonus wäre mit Geweben unter normalen Bedingungen nicht vergleichbar. Arteriolen sind sensitiv auf Sauerstoffmangel und reagieren mit einer Veränderung des Gefäßtonus. Bei einem intakten Organismus ist noch die Freisetzung von Katecholaminen zu erwarten, die ihrerseits den Gefäßtonus nochmals beeinflussen.

Tierexperimentelle Modelle haben generell, d.h. unabhängig von der Spezies, die ungünstige Eigenschaft, daß der Aufbau ihrer Haut von der des Menschen prinzipiell sehr verschieden ist. Es ist also keine vollständige anatomische Kompatibilität gegeben. Außerdem basiert die Pulsoximetrie auf stoffspezifischen Größen, den summierten spektralen Absorptionskoeffizienten der beiden Hämoglobinfraktionen Oxihämoglobin und De(s)oxihämoglobin. Da diese beiden Fraktionen von Spezies zu Spezies variieren, ist es verständlich, daß auch deren Absorptionskoeffizienten nicht identisch sind. So haben sogar adultes und fetales menschliches Hämoglobin geringfügig verschiedene Spektra. Wollte man die Kalibration also tierexperimentell angehen, müßte man erst die entsprechenden Hämoglobine spektral vermessen. Man erhält dann einen kalibrierten Tier-Pulsoximeter, der nur für die eine Spezies gültige Werte liefert. Der human-fetalen Kalibration kommt man so letztlich nicht näher. [Licht bedeutet in diesem Zusammenhang keineswegs nur sichtbares Licht, sondern weit darüber hinaus auch angrenzende Gebiete insbesondere Infrarot.]

Obwohl die Erfindung anhand der Kalibration eines Pulsoximeters beispielhaft beschrieben wird, ist sie hierauf nicht beschränkt, da das erfindungsgemäße Verfahren bzw. die erfindungsgemäße Vorrichtung selbstverständlich zur Messung sämtlicher Systeme herangezogen werden kann, bei welchen sich bestimmte lichtabsorbierende Parameter einer zu untersuchenden Körperflüssigkeit ändern und mittels Fotometrie gemessen werden können.

[Licht bedeutet in diesem Zusammenhang keineswegs nur sichtbares Licht, sondern weit darüber hinaus auch angrenzende Gebiete, insbesondere Infrarot]

Natürliche Gewebe eignen sich nicht sonderlich gut für so extrem niedrige Sauerstoff-Sättigungen, da es einerseits schwierig ist, das mit im System befindliche Gehirn vor dem Sauerstoffmangel zu schützen, andererseits, da die unphysiologisch niedrige Sauerstoff-Sättigung die Gewebe in vieler Hinsicht verändert.

Wir beschreiben daher die Erfindung eines künstlichen Gewebe-Modells, das jede Sättigung ohne Übergang von einem physiologischen in einen virtuellen pathologischen Bereich ermöglicht. Das Modell erfüllt folgende Eigenschaften:

- Es simuliert eine Arterien/Arteriolen-Pulsation im dem Sinne, daß zwischen Lichtsender und Lichtempfänger eine Distanzänderung erzeugt werden kann, deren Intensität (Amplitude) und Frequenz in großen Bereichen einstellbar ist.
- Es simuliert ferner den Arteriolen-Charakter in dem Sinne, daß pulsierendes, arterialisiertes Blut, d.h. Blut unmittelbar nach dem Gasaustausch in der Lunge, das Gewebemodell durchströmt. Damit kann prinzipiell jede Sauerstoff-Sättigung im Blut eingestellt werden.
- Es erlaubt die Anwendung des fetalen Transmissions-Pulsoximetrie-Sensors (und anderer [Reflexionssensoren- und Transmissions-] Pulsoximetrie-Sensoren) so, daß der Sensor vollständig und ausschließlich das arterielle Blut im Gewebemodell berücksichtigt.
- Es ermöglicht zudem eine exzellente Reproduzierbarkeit und Genauigkeit (Sauerstoff-Sättigung, Herzfrequenz, Pulsform, Pulsoberwelligkeit), wie sie mit natürlichen Geweben nie erreichbar wäre. Von besonderem Interesse ist eine sinusförmige optische Plethysmographie und die daraus resultierende Reinheit des Frequenzspektrums.
- Es macht unabhängig von biologischen Eigenschaften natürlicher Gewebe wie Ödeme, Hämatome, Verletzungen. Es macht ferner unabhängig von physiologischen Größen wie Blutdruck, Herzfrequenz, Blutfluß. Jede der Größen kann unabhängig eingestellt und konstant gehalten werden.
- Es umgeht die Notwendigkeit, über die Lunge bestimmte (hirngefährdende) Sauerstoff-Sättigungen einzustellen, d.h. ein steady state durch Einatmen eines definiert zusammengesetzten Gasgemisches (Stickstoff, Sauerstoff, Kohlendioxid) zu erzeugen.
- Es macht unabhängig von Bewegungsartefakten, von Fremdlicht, Temperaturschwankungen und eingeschränkter Perfusion (Schock).
- Es ermöglicht die notwendige Transparenz und Nachprüfbarkeit bei den Zulassungstellen.
- Es ermöglicht, jede beliebige Spezies zu imitieren/simulieren indem mit dem speziesspezifischen Blut (Hämoglobin) gearbeitet wird.

## Simulation von arteriell durchblutetem Gewebe und/oder Störungen zur Analyse von Pulsoxymetriegeräten mit deren Elektronik, Software und Sensoren

Bei der Pulsoximetrie werden üblicherweise zwei oder mehrere Leuchtdioden (LEDs) wechselseitig nacheinander periodisch eingeschaltet und damit die Transmission und/oder Reflexion des ausgestrahlten Lichtes durch das arteriell durchblutete Gewebe ermittelt. Bei der Transmissionspulsoximetrie durchstrahlt Licht von mindestens zwei Wellenlängen das arteriell durchblutete Gewebe und gelangt dann auf einen Fotoempfänger. Erfindungsgemäß wird das Gewebe durch eine lichtundurchlässige Platte ersetzt, so daß kein Licht von den Leuchtdioden zum Fotoempfänger gelangt. Auf dieser Trennplatte befinden sich in Richtung der LED-Seite des Sensors mindestens zwei Fotodioden und in Richtung der Fotodiode des Sensors eine Leuchtdiode oder eine gleichwertige Simulationseinrichtung für Reflexions-Pulsoximetrie-Sensoren.

Funktionsablauf:

Wenn eine der Sensor-LEDs aufleuchtet, empfängt eine Fotodiode des Trendplattenanalysators das ungefilterte Licht und wandelt es in geeigneter Form in ein elektrisches Signal um. Die zweite Fotodiode empfängt gefiltertes Licht derart, daß mit ihrem elektrischen Signal die sendende Sensor-LED identifiziert werden kann (Koinzidenz-, Ausschlußkriterien). Z.B.: Ist ein infrarotes Filter vor dieser zweiten Fotodiode angeordnet, kann diese nur die infrarote LED sehen, rote Licht wird dagegen so stark gedämpft, daß keine ausreichende Lichtleistung diese Fotodiode erreicht. Mit dieser Anordnung (ungefiltertes und gefiltertes Licht) kann man erkennen wie groß die Lichtamplitude ist und welche der LEDs leuchtet. Mit mehreren optischen Filtern und Fotodioden sind auch mehr als zwei LEDs zu unterscheiden. Das elektrische Signal der ungefilterten Fotodiode wird erfindungsgemäß logisch und/oder physikalisch in zwei Teilsignale aufgespalten, die proportional den Lichtamplituden der Sensor-LEDs sind.

Omega=(ACrot/DCrot)/(ACir/DCir)

vollständig simuliert werden. Die Omega-Gleichung bildet in den meisten Pulsoximetern die Grundlage bzw. Zwischengröße zur Bildung des angezeigten $SaO_2$-Wertes. Die "AC-Werte" können in geeigneter Weise verändert werden, so daß Herzschlag, Durchblutung,
Fingerdicke, Bewegungsartefakte, Rauschen und andere Störungen beliebig simuliert werden.

Mit diesem Trennplattensimulator kann nur sehr exakt und variabel ein Pulsoximeter getestet werden:

- Endkontrolle eines Pulsoximeter-Herstellers
- Überprüfung bei Zulassungsbehörden
- Genaue Funktionstests bei wiederkehrenden Prüfungen beim Anwender

Eigenschaften:

- Trennung des Lichtwegs
- Empfangen der Lichtamplitude
- Erkennen, welche LED leuchtet
- Multiplikatoren für jede einzelne LED-Amplitude
- Multiplikatoren so ausgewählt, daß Grundabsorption, Herzschlag, Durchblutung, Fingerdicke, Bewegungsartefakte, Rauschen, Störungen und andere Merkmale simuliert werden können
- Generieren eines Lichtsignals mit ein oder mehreren LEDs, das proportional dem Eingangslicht und den jeweiligen Multiplikatoren ist

Somit ist die Ansteuerung der Sensor-LED bekannt. Es kann nunmehr die Trennplatten-LED so angesteuert werden, daß die Sensor-Fotodiode.ein Signal erhält, als befände sich ein pulsatil durchblutetes Gewebe darin. Dazu müssen die beiden analysierten Sensor-LED-Funktionen mit virtuellen optischen Eigenschaften eines arteriell durchbluteten Gewebes multipliziert werden. Aus der Synthese der Sensor-LED-Ansteuerung an Seite des Pulsoximeters und der aufmodulierten Gewebeeigenschaften entsteht ein Signal, das von der Trennplatten-LED an die Fotodiode des Pulsoximeters übergeben wird. Im Einzelnen können folgende Merkmale simuliert werden:

- Grundabsorption
- Herzschlag
- Durchblutung
- Fingerdicke
- Bewegungsartefakte
- Rauschen
- Störungen
- andere Merkmale

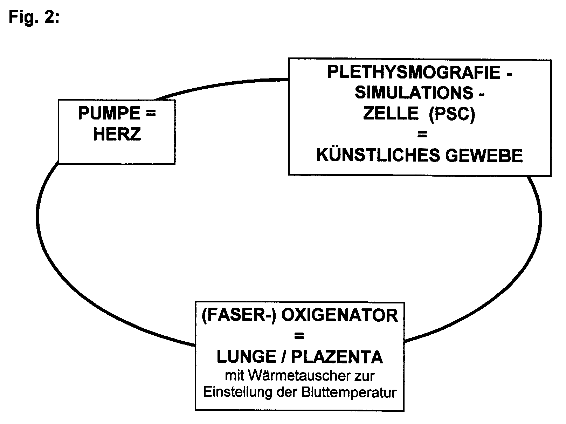

Folgende sind die Kernelemente des erfindungsgemäßen Kalibrationsaufbaus (s. Fig.2):

**1. PUMPE = HERZ:**
Als künstliches Herz wurde eine Rollerpumpe vorgesehen. Mit ihr wurde Blut in einem Kreislauf aus Pumpe, Oxigenator und künstlichem Gewebe entsprechend Herz, Lunge und Gewebe umgepumpt.

**2. OXIGENATOR = LUNGE:**
Als künstliche Lunge dient prinzipiell ein System, das Blut mit Gasen äquilibrieren kann. Als eine mögliche Ausführung wurde ein Faser-Oxigenator verwendet (Firma Terumo, Type CAPIOX 308), der das Blut mit einem Luft bzw. Sauerstoff/Stickstoff-Kohlendioxid-Gasgemisch äquilibriert, dessen Zusammensetzung völlig frei gewählt werden kann. Tausende von dünnen, porösen Kunststoffschläuchen führen das Blut von einem Ende zum anderen, während es von dem Gasgemisch umgeben ist. Durch die Poren ist ein leichter Austausch möglich. Der Oxigenator enthält zusätzlich einen Wärmetauscher, der von 37°C warmem Wasser durchflossen wird, um das Blut auf der venösen Seite auf Körpertemperatur zu bringen, damit dann die Sauerstoffaufnahme unter den physiologisch korrekten Bedingungen erfolgen kann. Auch andere Temperaturen können analog realisiert werden, damit auch die Kalibration für eine temperaturabhängige Absorption des Blutes durchgeführt werden kann.

**3. PLETHYSMOGRAPHIE-SIMULATIONS-ZELLE = KÜNSTLICHES GEWEBE:**
Das Kernstück der Erfindung ist das künstliche Gewebe. Es besteht im Prinzip aus einem Behälter, der in den Kreislauf integriert ist und durch den also Blut fließt, das direkt aus dem Oxigenator kommt. In dem Behälter ist ein Pulsoximetrie-Sensor fest montiert. Eine geeignete Vorrichtung bewirkt, daß sich entweder der mittlere effektive Lichtweg zwischen dem Lichtsender und dem Lichtempfänger oder die Absorptionsschichtdicke rhythmisch verändert, etwa mit der Frequenz, evtl. auch mit der Pulsform eines typischen plethysmografischen Signals. Prinzipiell ist nahezu jede Signalform möglich, insbesondere ist eine Sinusschwingung wegen ihrer spektralen Reinheit vorzuziehen. Der Pulsoximetriesensor befindet sich ganz oder zumindest so weit in Blut wie es notwendig ist, um die Lichtstrecke zwischen Sender und Empfänger vollständig und inklusive der streuungsbedingten Umwege durch Blut gehen zu lassen. In dieser Anordnung erzeugt jede der pulsatilen Distanzänderungen bzw. Änderungen der Absorptionsschichtdicke eine Änderung der Transmission gemäß dem Gesetz von Lambert und Beer, das die Verhältnisse recht gut beschreibt, obwohl viele seiner Voraussetzungen, insbesondere das Fehlen jeglicher Streuung, nicht erfüllt sind. Wie in der Pulsoximetrie üblich, vermißt man diese Transmissionsänderung in beiden (evtl. auch einmal mehreren Wellenlängen, um noch mehr Hämoglobin-Fraktionen oder andere relevante Farbstoffe in ihrer relativen Konzentration zu bestimmen) Hämoglobin-Fraktionen. Die Meßwerte werden dann elektronisch erfaßt, letztlich irgendwann in zwei Kanäle getrennt, analog -> digital gewandelt und bestimmte Werte gebildet, insbesondere eine Zwischengröße, die in der Literatur meist Omega genannt wird. Die genaue mathematische Ableitung folgt weiter unten. Das Prinzip des erfindungsgemäßen Gewebemodells ist eine mechanische, mittlere effektive Lichtwegsänderung zwischen Lichtsender und Lichtempfänger in bzw. unter Blut, dessen Sauerstoff-Sättigung eingestellt werden kann, bzw. untersucht werden soll. **Pulsationen des mittleren effektiven Lichtweges Sender -> Empfänger bzw. Pulsationen der werden nicht durch Blutdruckamplituden erzeugt, sondern der SENSOR BEFINDET SICH IM BLUT und der MITTLERE EFFEKTIVE LICHTWEG BZW. DIE EFFEKTIVE ABSORPTIONSSCHICHTDICKE WIRD DIREKT MECHANISCH PULSATIL VERÄNDERT. Konsequenz:**

- **VOLL GÜLTIGE WERTE**
- **KEINE EINSCHRÄNKUNGEN DURCH UNVERTRÄGLICHKEIT NIEDRIGER SAUERSTOFF-SÄTTIGUNGEN BZW. DURCH BEWEGUNGSARTEFAKTE.**

Das Gewebemodell besteht in einer möglichen, erfindungsgemäßen Ausführung aus einer Kammer (z.B. aus Metall) mit einem abnehmbaren Deckel. Eine Seite der Kammer bildet ein großer Topfmagnet, der über einen praktisch nicht dehnbaren Faden im Inneren der Kammer die mittlere effektive Lichtwegsänderung und damit auch die Transmissionsänderung bewirkt. Beim fetalen Transmissions-Pulsoximetrie-Sensor (Bild siehe Abbildung) bietet sich an, die Spiralfeder selbst als Rückstellkraft einzusetzen und die Feder einfach rhythmisch auseinanderzuziehen. Wird der Magnet wieder stromlos, so zieht sich die Spirale wieder zusammen und nimmt den Faden und den bewegten Magnetanker (oder eine Topfspule) mit zurück. Es gibt eine Reihe verschiedener Gründe, das Blutvolumen möglichst gering zu halten: Fetalblut steht nur in kleinen Mengen zur Verfügung, insbesondere wenn es als Nabelschnur-Einschlußblut gewonnen wurde. Außerdem sind kleine Blutmengen weniger träge, sowohl im Wärmetauscher, als auch beim Äquilibrieren mit Gasen.

- In einer weiteren, erfindungsgemäßen Ausführung erfolgt die Änderung der Absorption durch periodisches Einbringen eines transparenten Körpers, z.B. eines Glaskeils oder einer Glasstufe, die Blut verdrängt. So kommt eine Blutschichtdickenveränderung zustande. Ein Servomotor mit wechselnder Drehrichtung und einer Gewindespindel zur Umsetzung der Rotationsbewegung in eine Translationsbewegung bewegt den optischen Keil zwischen Lichtsender und Lichtempfänger. Dieses Kalibrationsprinzip kann so verwendet werden bei fetalen Transmissions-Pulsoximetrie-Sensoren bei denen sowohl Licht-Emitter- als auch Lichtempfänger innerhalb des Gewebes zu liegen kommen d.h. mit der Spirale eingestochen werden. Diese sogenannten inside-inside-Sensoren können folgendermaßen aufgebaut sein: In dem Teil der Spirale, der im applizierten Zustand im Gewebe zu liegen kommt, sind zwei einander gegenüber liegende Fenster aus der Spirale herausgearbeitet. In einem Fenster sind die beiden LEDs angeordnet, im anderen ist eine kleine Fotodiode plaziert.

- Das gesamte System ist ein künstlicher Kreislauf, d.h. die Komponenten sind analog ihrer Stellung im Organismus im Kreis angeordnet. Das Blut wird also im Kreis(lauf) (um-)gepumpt.

THEORETISCH MÖGLICHE METHODEN DER KALIBRATION:

Vergleich der vom Pulsoximeter angezeigten Sauerstoff-Sättigung mit einer Referenz

| GEWEBEMODELL | - SÄTTIGUNGS - BEREICH | BEMERKUNGEN |
| --- | --- | --- |
| Echtes Gewebe mit Hirn (Mensch) | (50) 60 - 100 % | arterielle Blutproben, von Probanden im steady state, die Gemische von $O_2$ und $N_2$ atmen |
| Echtes Gewebe mit Hirn (Tier) | (0) 50 - 100 % | Geht davon aus, daß der Referenz-Pulsoximeter korrekte Werte anzeigt (im steady state) |

(fortgesetzt)

| GEWEBEMODELL | - SÄTTIGUNGS - BEREICH | BEMERKUNGEN |
|---|---|---|
| Echtes Gewebe ohne Hirn (Tier) | (0) 50 - 100% | Prinzipiell gut, aber Einschränkungen echten Gewebes (artifizielle Sauerstoff-Sättigung) |
| Echtes Gewebe ohne Hirn (Mensch) z.B. Anencephalie, Hirntoter, postmortal | (0) 50- 100% | Ethische Problematik Wissenschaftlich bestes Modell mit echtem Gewebe! Einschränkungen echten Gewebes (artifizielle Sauerstoff-Sättigung) |
| Plethysmographische Simulation mit Blut (Exzitation eines Pulsoximetrie-Sensors mit pulsierendem, blutgefülltem Schlauch) | 0 - 100% | Der pulsierende Blutdruck selbst dehnt auf, d.h. vergrößert den mittleren effektiven Lichtweg Emitter -> Sensor. Unvermeidbare und unkontrollierbare Schlauchbewegungen erzeugen Bewegungsartefakte |
| Nichtplethysmographische Simulation ohne Blut (andere Farbstoffe) | ca. 50 - 100 % (in der Literatur beschrieben) | nur bedingt aussagefähig, Farbstoff nicht Hämoglobin, für jede simulierte Sättigung ist ein eigenes System nötig, Pulsfrequenz und -form schlecht einstellbar |
| **PLETHYSMOGRAPHIE- SIMULATION MIT BLUT (jedweder Provenienz): PLETHYSMOGRAPHI E- SIMULATIONS- ZELLE = KÜNSTLICHES GEWEBE** <br><br> **= ERFINDUNG** | **0 - 100 % voller Bereich nutzbar. Zur Überprüfung der biologischen Validität der Meßwerte werden die Werte im physiologischen Bereich mit Werten verglichen, die an Probanden gemessen wurden.** | **Pulsationen des effektiven Lichtwegs zwischen Sender und Empfänger werden nicht durch Blutdruckamplituden erzeugt, sondern der SENSOR IST IM BLUT und der EFFEKTIVE LICHTWEG WIRD DIREKT MECHANISCH PULSATIL VERÄNDERT. Konsequenz: VOLL GÜLTIGE WERTE KEINE EINSCHRÄNKUNGEN DURCH UNVERTRÄGLICHKEIT NIEDRIGER SAUERSTOFF- SÄTTIGUNG BZW. BEWEGUNGSARTEFAKTE** |

Die Pulsoximetrie ist eine kontinuierliche Fotometrie lebenden, arteriell, d.h. pulsatil durchbluteten Gewebes in typischerweise zwei Spektralbereichen. Sie beruht auf der Tatsache, daß Hämoglobin in zwei Fraktionen vorliegt, als oxigeniertes Hämoglobin und als deoxigeniertes Hämoglobin. Diese beiden Hämoglobinfraktionen unterscheiden sich in ihrer spezifischen spektralen Absorption, sind also zwei verschiedene Farbstoffe. Man kennt dies aus der Beobachtung, daß z.B. Venen oder Blutergüsse blau erscheinen, sauerstoffarmes Hämoglobin also blau wirkt, gut mit Sauerstoff versorgtes, z.B. Sonnenbrand, Entzündungen dagegen rot.

Für die Pulsoximetrie wird Gewebe durchstrahlt, die absolute Schichtdicke, z.B. die Dicke eines Fingers ist aber nicht bekannt. Die erhaltene Sauerstoffinformation wird daher nicht absolut (z.B. in Gramm pro Liter) sein, sondern relativ: Man erhält die prozentuale Sauerstoff-Sättigung, das ist der Anteil oxigenierten Hämoglobins am Gesamthämoglobin. Unter Gesamthämoglobin wird in diesem Zusammenhang die Summe aller meßbaren Hämoglobine verstanden, das sind bei nur zwei Spektralbereichen die zwei Fraktionen: oxigeniertes Hämoglobin und deoxigeniertes Hämoglobin. Liegen keine anderen Hämoglobinfraktionen in nennenswertem Ausmaß vor, so mißt die Pulsoximetrie zuverlässige Werte. Liegen aber z.B. Methämoglobin oder Carboxihämoglobin in höheren Konzentrationen vor, so entstehen systemimmanente Fehler.

Pulsoximetrie gibt es prinzipiell in zwei Varianten, die Transmissionspulsoximetrie und die Reflexionspulsoximetrie, je nach vorwiegendem Strahlengang. Werden Lichtsender und Lichtempfänger durch das Gewebe optisch nahezu vollständig voneinander getrennt, befindet sich also das Gewebe mehr oder weniger in der Mitte zwischen Sender und Empfänger, so spricht man von Transmissionspulsoximetrie. Von Reflexpulsoximetrie spricht man, wenn Sender und Empfänger im wesentlichen auf der gleichen Seite des Gewebes außen auf der Haut angeordnet sind und im Sender erzeugtes Licht direkt oder indirekt den Empfänger erreichen kann. Es ist ein wesentliches Merkmal der Reflexpulsoximetrie, daß auch Licht den Empfänger erreicht, das das Gewebe nicht wirklich durchstrahlt hat, sondern im Sinne eines optischen Shunts (Kurzschlusses) den Empfänger erreicht (indem es z.B. in den obersten Hornhautschichten fortgeleitet wird). Wie diese Definition vermuten läßt, sind fließende Übergänge möglich, wenn durch spezielle Maßnahmen der Anteil an Shunt-Licht klein wird.

Die Pulsoximetrie mißt die arterielle Sauerstoff-Sättigung. Sauerstoff-Sättigung ist definiert als der Anteil von oxigeniertem Hämoglobin am Gesamthämoglobin:

$$\text{Sauerstoffsättigung} = \frac{\text{oxigeniertes Hämoglobin}}{\text{Gesamthämoglobin}} = \frac{Hb_{O2}}{Hb_{ges}}$$

Prinzipiell doppelsinnig kann der Begriff "Gesamthämoglobin" interpretiert werden. Je nachdem, ob man darunter lediglich die Summe aus oxigeniertem Hämoglobin und deoxigeniertem Hämoglobin versteht, führt dies natürlich zu einer anderen Sauerstoffsättigung, als wenn man noch weitere eventuell vorkommende Hämoglobinfraktionen mit in die Summe einbezieht. Man hat zur besseren Unterscheidung zwei Begriffe gebildet:

- fraktionelle Sauerstoff-Sättigung und
- funktionelle Sauerstoff-Sättigung.

Die korrekteste Definition bezieht die Konzentration von oxigeniertem Hämoglobin auf die Summe aller denkbaren Hämoglobin-Fraktionen als Gesamthämoglobin:

$$\text{fraktionale Sauerstoffsättigung} = \frac{\text{oxigeniertes Hämoglobin}}{\text{Gesamthämoglobin}}$$

$$= \frac{Hb_{O2}}{Hb_{red} + Hb_{O2} + Hb_{Met} + Hb_{CO} + Hb_{S} + Hb...}$$

Aus praktischen Gründen ist es sinnvoll, sich auf ein Gesamthämoglobin zu beschränken, das man sich aus nur zwei Komponenten bestehend vorstellt: den beiden optisch mit zwei Wellenlängen meßbaren Hämoglobin-Fraktionen, oxigeniertem Hämoglobin und deoxigeniertem Hämoglobin (Zwei-Wellenlängen-Pulsoximetrie):

$$\text{funktionale Sauerstoffsättigung} = \frac{\text{oxigeniertes Hämoglobin}}{\text{oxigeniertes Hämoglobin} + \text{deoxigeniertes Hämoglobin}}$$

$$= \frac{Hb_{O2}}{Hb_{red} + Hb_{O2}}$$

Die aussagefähigste Sättigungsdefinition ist sicherlich die **fraktionelle Sauerstoff-Sättigung.** Dennoch ist die von Pulsoximetern bestimmte funktionelle Sauerstoff-Sättigung klinisch sehr brauchbar (wenn man von den letztlich seltenen und/oder geringgradigen Dyshämoglobinämien absieht) . Für didaktische Zwecke überspitzt wurde diese Problematik öfter so ausgedrückt: Die Pulsoximetrie kann entweder die richtige Sauerstoff-Sättigung falsch oder die falsche Sauerstoff-Sättigung richtig messen. Man hat sich entschieden die falsche Sauerstoff-Sättigung richtig zu messen, da sie doch in den meisten Fällen klinisch relevante Ergebnisse liefert.

Theoretisch wäre es möglich mittels mehrerer enger Spektralbereiche noch weitere Hämoglobinfraktionen meßbar zu machen (Multi-Wellenlängen-Pulsoximetrie). Pulsoximeter müssen meist nicht vom Anwender kalibriert werden, lediglich einmal werden sie herstellerseitig kalibriert.

Unter Kalibration versteht man die Herstellung eines Zusammenhanges zwischen der vom Pulsoximeter berechneten Meßgröße Omega (meist in der Literatur verwendet, Definition siehe unten) und der wirklich im untersuchten Gewebe vorliegenden Sauerstoff-Sättigung. Letztere muß mit einer Methodik bestimmt werden, deren Meßgenauigkeit bekannt und ausreichend ist, d.h. die die des zu kalibrierenden Pulsoximeters deutlich übersteigt. Hierzu finden meist sog. CO-Oximeter Verwendung (z.B. OSM3 von Radiometer oder ABL 270 von Ciba Geigy Corning), mit deren Hilfe man die Sauerstoff-Sättigung von Blutproben direkt bestimmen kann. Prinzipiell kommen auch Blutgasanalysatoren zur Bestimmung der Sauerstoff-Sättigung aus Blutproben in Frage, aber sie berechnen die Sauerstoff-Sättigung aus verschiedenen gemessenen Größen (Partialdrucken) unter Verwendung einiger Hypothesen. Naturgemäß ist die zu erwartende Genauigkeit einer direkt gemessenen Größe höher als die einer aus indirekt gemessenen Größen errechneten. Um einen Pulsoximeter mit einem CO-Oximeter zu kalibrieren muß man ein Gewebe haben, an dem man Pulsoximetrie anwenden kann, z. B. einen Finger. Es werden dann innerhalb eines sinnvollen und ethisch vertretbaren Bereichs durch Atmung von Stickstoff-Sauerstoffgemischen mit definierter Sauerstoffkonzentration stabile Sauerstoff-Sättigungen im arteriellen Blut erzeugt, so daß diese Sauerstoff-Sättigungen, die mit Blutproben am CO-Oximeter gewonnen werden, dem zu kalibrierenden Pulsoximeter zugeordnet werden können. Es ergibt sich so ein Zusammenhang zwischen einer bekannten Sauerstoff-Sättigung und einer zu bestimmenden. Es ist das Ziel dieser Messungen den Pulsoximeter so zu kalibrieren, daß er die wirklichen Verhältnisse (bezogen auf den Standard CO-Oximeter) wiedergibt.

Das durchstrahlte Gewebe kann man sich aus mehreren Schichten zusammengesetzt denken, von denen jede zur Gesamtabsorption beiträgt. Das nebenstehende Schema greift die wichtigsten Schichten heraus.

pulsierendes arterielles Blut → 

- Systole
- Diastole

| arterielles Blut (konstanter Teil) |
| kapilläres Blut |
| venöses Blut |
| Knochen |
| Gewebe (Muskel, Bindegewebe, Fett) |
| (evtl. pigmentierte) Haut |

Das Lambert-Beer'sche Gesetz beschreibt die Absorption Von Licht durch ein absorbierendes Medium. Obwohl einige Grundvoraussetzungen für dieses Gesetz nicht erfüllt sind (monochromatisches Licht, geringe Konzentration, keine Streuung), lassen sich die realen Absorptionsverhältnisse in der Pulsoximetrie erstaunlich gut beschreiben. Für die zusätzliche Absorption des Gewebes bei jedem Herzschlag ist eine Schichtdickenzunahme des Gewebes verantwortlich, hervorgerufen durch zusätzlich einströmendes arterielles Blut und eine Ausdehnung der **Arteriolen**. Die anatomisch verantwortliche Struktur für die Pulsoximetrie sind also die Arteriolen.

$$I = Io * e^{-\varepsilon * c * d}$$

mit:

I = Intensität des Lichtes nach Durchlaufen der Schichtdicke d
Io = Intensität des eingestrahlten Lichtes
c = Konzentration des Farbstoffes (wellenlängenunabhängig)
d = Schichtdicke des absorbierenden Farbstoffs (wellenlängenabhängig durch Streuung)
$\varepsilon$ = spezifischer Absorptionskoeffizient (gültig nur für eine bestimmte Wellenlänge)

Eine pulsatile Schichtdickenzunahme kann folgendermaßen ausgedrückt werden:

$$I = Io * e^{-\varepsilon * c(d+\delta)}$$

Ergebnis: Es ist wichtig zu verstehen, daß sowohl die konstante Absorptionsstrecke (d) als auch die pulsatile Absorptionsstrecke $\delta$ das Blut enthalten müssen, dessen Sauerstoff-Sättigung bestimmt werden soll, also das arterielle bzw. besser das arterialisierte Blut.

Es kommen hierfür prinzipiell drei Alternativen in Frage:

1. Echtes menschliches Gewebe (Probanden, Patienten) eines intakten, lebensfähigen Organismus normal, d.h. durch ein vitales Herz perfundiert,
2. Echtes menschliches Gewebe eines letztlich nicht lebensfähigen Organismus (Hirntod, Anencephalie) normal, d.h. durch ein intaktes Herz perfundiert, evtl. auch künstlich perfundiert (Herz-Lungen-Maschine). Ethik ?
3. Tierisches Gewebe,

a) vollständiges lebendes Tier, natürlich perfundiert (Herz) bzw.
b) vollständiges totes bzw. anästhesiertes Tier, künstlich perfundiert (tierexperimentelle Herz-Lungen-Maschine)
c) Körperteil oder Organ eines Tieres, dessen Gefäße gut zugänglich sind so, daß es künstlich perfundiert (tierexperimentelle Herz-Lungen-Maschine) und von Blut durchströmt werden kann.

4. Künstliches Gewebe, d.h. Gewebemodell für die Pulsoximetrie, das gewissermaßen als Minimalforderung die wesentlichen Eigenschaften von Arteriolen besitzen muß, da Arteriolen das anatomische Substrat der Gewebspulsatilität und damit verantwortlich für die optische Plethysmographie sind. Das künstliche Gewebe-Modell muß also wenigstens folgende Eigenschaften bzw. Komponenten aufweisen:

a) einen Hohlraum,

- der von arterialisiertem Blut, d.h. Blut definierter Sauerstoff-Sättigung durchströmt werden kann
- der in mindestens einer Dimension optisch transluzent oder transparent ist
- und dessen Abmessungen, wie sich dehnende Arteriolen, pulsatil veränderlich sind.

In Einhaltung dieser Eigenschaften sind bisher verschiedene künstliche Gewebe vorgeschlagen worden. Zur Untersuchung von Fingersensoren wurde zum Beispiel eine Art Schlauch angegeben, der, mit Blut gefüllt, im Inneren des Fingersensors zu liegen kommt. Werden Druckschwankungen im Schlauch erzeugt, so entsteht eine Änderung des Schlauchdurchmessers. Diese Schichtdickenänderung wird vom angeschlossenen Pulsoximeter ebenfalls registriert und als optisch plethysmographisches Signal gedeutet. Es wird so die Sauerstoff-Sättigung der veränderlichen Blutschicht bestimmt, das ist bei dem vorliegenden künstlichen Gewebe die Sauerstoff-Sättigung der gesamten durchstrahlten Blutschicht. Es wurden noch andere Vorschläge gemacht, um veränderliche Blutschichtdicken zu erzeugen.

**Physikalische Gesetze und mathematische Ableitungen**

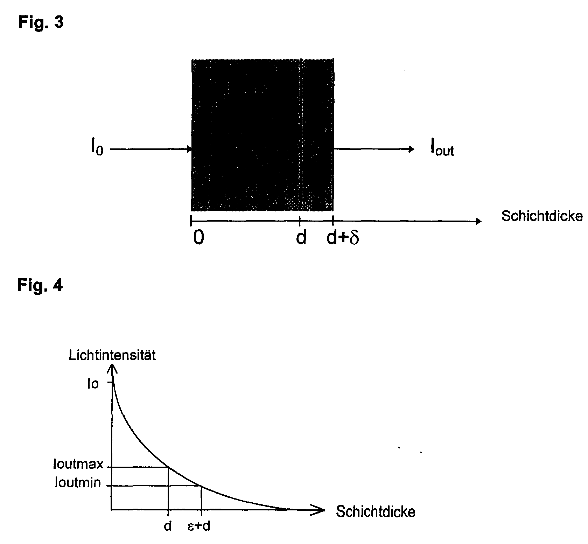

In der folgenden mathematischen Ableitung wird auf die Figuren 3 und 4 Bezug genommen. Die verwendeten Abkürzungen und Bezeichnungen haben folgende Bedeutungen:

Io = einfallende Lichtintensität
Iout = austretende Lichtintensität
c = Konzentration des absorbierenden Mediums
$\varepsilon$ = Extinktionskoeffizient des absorbierenden Mediums
d = Schichtdicke
$\delta$ = zusätzliche Schichtdicke

$$I_{out\,max} = I_o * e^{\varepsilon * c * d}$$

$$I_{out\,min} = I_o * e^{\varepsilon * c * (d+\delta)}$$

Bezieht man die austretende Lichtintensität ($I_{out}$) auf die einfallende Lichtintensität ($I_o$), dann erhält man die relative Lichtabschwächung:

$$\frac{I_{outmax}}{Io} = e^{\varepsilon * c * d}$$

$$\frac{I_{outmin}}{Io} = e^{\varepsilon * c * (d+\delta)}$$

Werden nun der Quotient der relativen Lichtabschwächungen jeweils mit der Dicke d und der Dicke d+$\delta$ gebildet:

$$\frac{\dfrac{I_{out\ max}}{Io}}{\dfrac{I_{out\ min}}{Io}} = \frac{e^{-\varepsilon * c * d}}{e^{-\varepsilon * c * (d+\delta)}}$$

$$\frac{I_{out\ max}}{I_{out\ min}} = e^{-\varepsilon * c * d + \varepsilon * c * (d+\delta)}$$

$$\frac{I_{out\ max}}{I_{out\ min}} = e^{+\varepsilon * c * \delta}$$

durch Logarithmieren beider Seiten der Gleichung:

$$\ln\left(\frac{I_{out\ max}}{I_{out\ min}}\right) = = \ln\left(e^{+\varepsilon * c * \delta}\right)$$

$$\ln\left(\frac{I_{out\ max}}{I_{out\ min}}\right) = = \varepsilon * c * \delta$$

Nach dieser Einführung betrachten wir ein System mit mehreren Extinktionskoeffizienten und mit variablen und konstanten Schichtdicken.

$$I_{out\ min\ 1} = I_o * e^{-\varepsilon 1 * c 1 * d 1}$$

$$I_{out\ min\ 2} = I_{out\ min\ 1} * e^{-\varepsilon 2 * c 2 * d 2}$$

$$I_{out\ min} = I_{out\ min\ 2} * e^{-\varepsilon 1 * c 1 * \delta 1}$$

$$I_{out\ min} = I_o * e^{-\varepsilon 1 * c 1 * d 1} * e^{-\varepsilon 2 * c 2 * d 2} * e^{-\varepsilon 1 * c 1 * \delta 1}$$

$$I_{out\ min} = I_o * e^{-(\varepsilon 1 * c 1 * d 1 + \varepsilon 2 * c 2 * d 2 + \varepsilon 1 * c 1 * \delta 1)}$$

$$\frac{I_{outmin}}{Io} = e^{-(\varepsilon1*c1*d1+\varepsilon2*c2*d2+\varepsilon1*c1*\delta1)}$$

analog erhält man für $I_{out\,max}$ :

$$I_{out\,max} = I_o * e^{-\varepsilon1*c1*d1} * e^{-\varepsilon2*c2*d2}$$

$$I_{out\,max} = I_o * e^{-(\varepsilon1*c1*d1+\varepsilon2*c2*d2)}$$

$$\frac{I_{outmax}}{Io} = e^{-(\varepsilon1*c1*d1+\varepsilon2*c2*d2)}$$

durch Quotientenbildung von $\frac{I_{outmax}}{I_{out\,min}}$ erhält man

$$\frac{I_{outmax}}{I_{out\,min}} = \frac{e^{-(\varepsilon1*c1*d1+\varepsilon2*c2*d2)}}{e^{-(\varepsilon1*c1*d1+\varepsilon2*c2*d2+\varepsilon1*c1*\delta1)}}$$

$$\frac{I_{outmax}}{I_{out\,min}} = e^{-(\varepsilon1*c1+d1+\varepsilon2*c2*d2)+(\varepsilon1*c1*d1+\varepsilon2*c2*d2+\varepsilon1*c1*\delta1)}$$

$$\frac{I_{outmax}}{I_{out\,min}} = e^{+\varepsilon1*c1*\delta1}$$

durch Logarithmieren von beiden Seiten ergibt sich

$$\ln\left(\frac{I_{outmax}}{I_{out\,min}}\right) = \ln(e^{+\varepsilon1*c1*\delta1})$$

$$\ln\left(\frac{I_{outmax}}{I_{out\,min}}\right) = \varepsilon*c1*\delta1$$

Aus dieser Herleitung ist sofort erkennbar, daß alle nicht veränderten Schichtdicken und deren Extinktionskoeffizienten sowie Konzentrationen eliminiert wurden, ebenso wird durch diese Quotientenbildung

$$\ln\left(\frac{I_{outmax}}{I_{out\,min}}\right)$$

das absolute Eingangslicht eliminiert.

Wenn man auch mehrere Absorber mit unterschiedlichen Extinktionskoeffizienten einführt, gilt allgemein:

$$\ln\left(\frac{I_{out\,max}}{I_{out\,min}}\right) = \sum_{n} \varepsilon_n * c_n * \delta$$

Auf diesem fotometrischen Prinzip beruht die Pulsoximetrie. Bei dieser Art der Messung werden alle konstanten Schichten und somit auch deren Extinktionskoeffizienten wie z.B. Knochen, Gewebe, die absolute Meßstrecke (z.B. Fingerkuppe <--> Ohrläppchen) eliminiert. Nur der arteriell durchblutete Teil ist für den pulsatilen Teil der Transmission verantwortlich und somit ist die arterielle Sauerstoff-Sättigung bestimmbar.
(Eventuelle weitere pulsatile Anteile wie Venenpulsation oder andere Verdrängungseffekte können vernachlässigt werden.)

**Definition der funktionellen Sauerstoff-Sättigung** $SaO_{2func}$

$$SaO_{2func} = \frac{Hb_{oxi}}{Hb_{oxi} + Hb_{deoxi}}$$

mit $Hb_{oxi}$ für das oxigenierte und $Hb_{deoxi}$ für das reduzierte, deoxigeniertes Hämoglobin.
Bzw. mit den dazugehörigen Konzentrationen ausgedrückt:

$$SaO_{2func} = \frac{c_{oxi}}{c_{oxi} + c_{deoxi}}$$

Anschließend wird dir funktionelle Sauerstoff-Sättigung $SaO_{2func}$ zur späteren Verwendung nach $C_{deoxi}$ umgestellt:

$$c_{deoxi} = \frac{c_{oxi}}{SaO_{2func}} - c_{oxi}$$

$$c_{deoxi} = c_{oxi}\left(\frac{1}{SaO2func} - 1\right)$$

Führt man zwei Wellenlängen ein, dann erhält man:

$$\left[\varepsilon_{deoxi\lambda1} * c_{deoxi} + \varepsilon_{oxi\lambda1} * c_{oxi}\right] * \delta = \ln\left(\frac{I_{out\,max\,\lambda1}}{I_{out\,min\,\lambda1}}\right)$$

$$\left[\varepsilon_{deoxi\lambda2} * c_{deoxi} + \varepsilon_{oxi\lambda2} * c_{oxi}\right] * \delta = \ln\left(\frac{I_{out\,max\,\lambda2}}{I_{out\,min\,\lambda2}}\right)$$

$$\left[\varepsilon_{deoxi\lambda1} * \left(\frac{1}{SaO2func} - 1\right) + \varepsilon_{oxi\lambda1}\right] * c_{oxi} * \delta = \ln\left(\frac{I_{out\,max\,\lambda1}}{I_{out\,min\,\lambda1}}\right)$$

$$\left[\varepsilon_{deoxi\lambda2}*\left(\frac{1}{Sa_{O2func}}-1\right)+\varepsilon_{oxi\lambda2}\right]*C_{oxi}*\delta \qquad = \qquad \ln\left(\frac{I_{out\,max\,\lambda2}}{I_{out\,min\,\lambda2}}\right)$$

durch Quotientenbildung erhält man:

$$\frac{\left[\varepsilon_{deoxi\lambda1}*\left(\frac{1}{Sa_{O2func}}-1\right)+\varepsilon_{oxi\lambda1}\right]*C_{oxi}*\delta}{\left[\varepsilon_{deoxi\lambda2}*\left(\frac{1}{Sa_{O2func}}-1\right)+\varepsilon_{oxi\lambda2}\right]*C_{oxi}*\delta} \qquad = \qquad \frac{\ln\left(\frac{I_{out\,max\,\lambda1}}{I_{out\,min\,\lambda1}}\right)}{\ln\left(\frac{I_{out\,max\,\lambda2}}{I_{out\,min\,\lambda2}}\right)}$$

$$\frac{\varepsilon_{deoxi\lambda1}*\left(\frac{1}{Sa_{O2func}}-1\right)+\varepsilon_{oxi\lambda1}}{\varepsilon_{deoxi\lambda2}*\left(\frac{1}{Sa_{O2func}}-1\right)+\varepsilon_{oxi\lambda2}} \qquad = \qquad \frac{\ln\left(\frac{I_{out\,max\,\lambda1}}{I_{out\,min\,\lambda1}}\right)}{\ln\left(\frac{I_{out\,max\,\lambda2}}{I_{out\,min\,\lambda2}}\right)}$$

Durch diese Quotientenbildung wird die Schichtdickenänderung eliminiert. An dieser Stelle wird die Meßwertvariable $\Omega$ eingeführt:

$$\Omega = \frac{\ln\left(\frac{I_{out\,max\,\lambda1}}{I_{out\,min\,\lambda1}}\right)}{\ln\left(\frac{I_{out\,max\,\lambda2}}{I_{out\,min\,\lambda2}}\right)}$$

beziehungsweise

$$\Omega = \frac{\varepsilon_{deoxi\lambda1}*\left(\frac{1}{Sa_{O2func}}-1\right)+\varepsilon_{oxi\lambda1}}{\varepsilon_{deoxi\lambda2}*\left(\frac{1}{Sa_{O2func}}-1\right)+\varepsilon_{oxi\lambda2}}$$

diese Gleichung wird in mehreren Schritten nach $Sa_{O2}$ umgestellt.

$$\varepsilon_{deoxi\lambda1}*\left(\frac{1}{Sa_{O2func}}-1\right)+\varepsilon_{oxi\lambda1} \qquad = \qquad \Omega * \varepsilon_{deoxi\lambda2}*\left(\frac{1}{Sa_{O2func}}-1\right)+\varepsilon_{oxi\lambda2}$$

$$\frac{1}{Sa_{O2func}}\,[\varepsilon_{deoxi\lambda1} - \varepsilon_{deoxi\lambda2}*\Omega] = \Omega*[-\varepsilon_{deoxi\lambda2} + \varepsilon_{oxi\lambda2}]+\varepsilon_{deoxi\lambda1} - \varepsilon_{oxi\lambda1}$$

$$Sa_{O2func} = \frac{\varepsilon_{deoxi\lambda 1} - \varepsilon_{deoxi\lambda 2} * \Omega}{\Omega * [\varepsilon_{deoxi\lambda 2} - \varepsilon_{oxi\lambda 2}] + \varepsilon_{deoxi\lambda 1} - \varepsilon_{oxi\lambda 1}}$$

Somit wurde der rechnerische Zusammenhang der funktionellen Sauerstoff-Sättigung mit der Meßwertvariablen $\Omega$ mit zwei verschiedenen Lichtwellenlängen aufgezeigt.

Dieser strenge mathematische Zusammenhang gilt jedoch nur bei folgenden Voraussetzungen bzw. Einschränkungen:

- optisch homogenes Medium
- keine optische Streuung
- monochromatisches Licht
- Pulsation nur der zu messenden Medien, bzw. andere pulsatile Anteile vernachlässigbar

Wegen dieser strengen Anforderungen kann die funktionelle Sauerstoff-Sättigung praktisch nicht berechnet werden. Sie wird mit Hilfe einer empirisch ermittelten Kalibrationskurve bestimmt.

$$Sa_{O2} = f\left(\frac{\ln\left(\frac{I_{out\,max\,\lambda 1}}{I_{out\,min\,\lambda 1}}\right)}{\ln\left(\frac{I_{out\,max\,\lambda 2}}{I_{out\,min\,\lambda 2}}\right)}\right)$$

$$Sa_{O2} = f(\Omega)$$

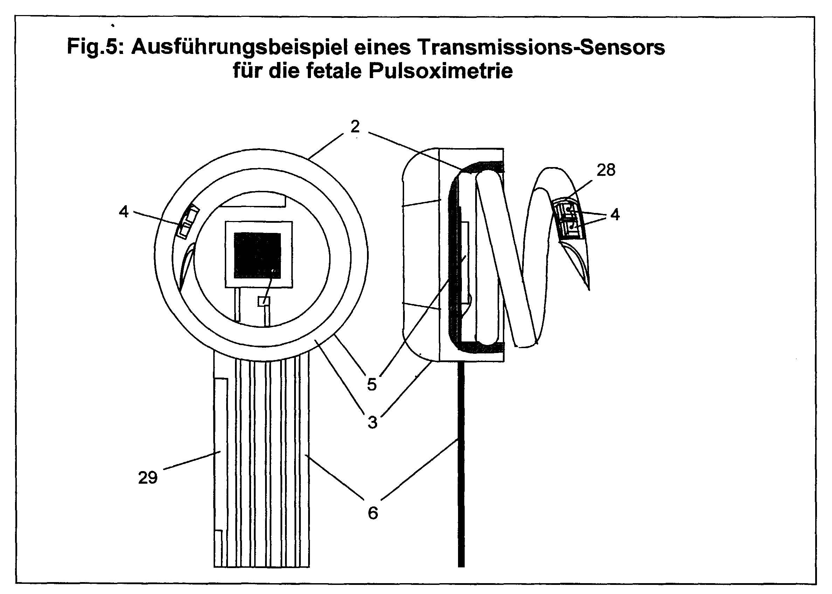

Diese empirische Kalibrationskurve berücksichtigt alle Abweichungen von den idealen Bedingungen für die Gültigkeit des Lambert-Beer'schen Gesetzes. Die Fig. 5 zeigt beispielhaft 3 Kalibrationskurven.

Anhang: In der Literatur wird häufig eine vereinfachte Gleichung zur Berechnung der Meßvariablen $\Omega$ angegeben. Sie leitet sich wie folgt her:

$$\Omega = \frac{\ln\left(\frac{I_{out\,max\,\lambda 1}}{I_{out\,min\,\lambda 1}}\right)}{\ln\left(\frac{I_{out\,max\,\lambda 2}}{I_{out\,min\,\lambda 2}}\right)}$$

$$\Omega = \frac{\ln\left(\frac{I_{out\,max\,\lambda 1}}{I_{out\,min\,\lambda 1}} - 1 + 1\right)}{\ln\left(\frac{I_{out\,max\,\lambda 2}}{I_{out\,min\,\lambda 2}} - 1 + 1\right)}$$

$$\Omega = \frac{\ln\left(\frac{I_{out\,max\,\lambda 1}}{I_{out\,min\,\lambda 1}} - \frac{I_{out\,min\,\lambda 1}}{I_{out\,min\,\lambda 1}} + 1\right)}{\ln\left(\frac{I_{out\,max\,\lambda 2}}{I_{out\,min\,\lambda 2}} - \frac{I_{out\,min\,\lambda 2}}{I_{out\,min\,\lambda 2}} + 1\right)}$$

$$\Omega = \frac{\ln\left(\dfrac{I_{out\ max\ \lambda1} - I_{out\ min\ \lambda1}}{I_{out\ min\ \lambda1}} + 1\right)}{\ln\left(\dfrac{I_{out\ max\ \lambda2} - I_{out\ min\ \lambda2}}{I_{out\ min\ \lambda2}} + 1\right)}$$

mit den Definitionen:     DC = Iout min

und               AC = Iout max- Iout min

wird:

$$\Omega = \frac{\ln\left(\dfrac{AC_{\lambda1}}{DC_{\lambda1}} + 1\right)}{\ln\left(\dfrac{AC_{\lambda2}}{DC_{\lambda2}} + 1\right)}$$

für x<<1     gilt     ln(x+1) = x

$$\Omega = \frac{\dfrac{AC_{\lambda1}}{DC_{\lambda1}}}{\dfrac{AC_{\lambda2}}{DC_{\lambda2}}}$$

Beispiel mit $\lambda1$= rotes Licht und $\lambda2$= infrarotes Licht gilt dann:

$$\Omega = \frac{\left(\dfrac{AC_{rot}}{DC_{rot}}\right)}{\left(\dfrac{AC_{infrarot}}{DC_{infrarot}}\right)}$$

Einzelheiten der Gewebe-SImulation:

Wie oben gezeigt, stellen Gewebe optische Wegstrecken dar, die zeitlich konstant oder veränderlich, sowohl Absorption als auch Streuung teilweise auch Reflexion aufweisen, abhängig von der spektralen Lage und Breite des Lichts. Einzelne Komponenten setzen sich zu einem gemeinsamen optischen Weg zusammen.

Blutleeres Gewebe (z.B. nach dem "Auswickeln" und anschließendem arteriellen Block oder nach spülender arterio-venöser Perfusion mit einer isotonen wässrigen Lösung) erscheint weißlich und streut das Licht unvermindert stark. Blut besteht nach dem Absetzen (Zentrifugation) zellulärer Bestandteile aus einer mehr oder weniger klaren und weitgehend farblosen wässrigen Lösung. Der zelluläre Anteil streut das Licht stark. Sowohl Gewebe als auch Blut (nicht entmischt) weisen also sowohl Absorption als auch Streuung auf. Beide, Streuung und Absorption sind wiederum abhängig von der Wellenlänge. Diese optischen Eigenschaften gelten sowohl für zeitlich veränderte optische Wege als auch für konstante. Wichtig ist, daß die optischen Eigenschaften verschiedener relevanter anatomischer Strukturen im Detail simuliert werden können:

A) Absorption und Streuung und Blutanteil (Anteil der Körperflüssigkeit) im zeitlich **unveränderlichen** Gewebe lassen sich wie folgt beeinflussen:

Blut/Hämoglobin trägt je nach Art und Konzentration (bzw. Konzentrationsverteilung der Hämoglobinkomponenten [z.B. Sauerstoff-Sättigung] erheblich zu Absorption und geringergradig auch zu Streuung im Gewebe bei. Man kann sich sowohl die Streuung als auch die Absorption blutgefüllten Gewebes also aus je zwei hintereinander-geschalteten Komponenten zusammengesetzt denken, die Reihenfolge spielt dabei keine wesentliche Rolle. Will man den Anteil des (blutleeren) Gewebes simulieren kann man ein weißes (farbneutrales) , streuendes Material, zum Beispiel ein Stück Milchglas einsetzen. Ideal ist es jedoch, die Streuwirkung unabhängig von der Schichtdicke zu verändern. Dazu kann man winzige Streuzentren z.B. kleine, einige Mikrometer große silanisierte Glaskugeln, ca. in der Größe von Erythrozyten in Gießharz eindispergiert. Sie entfalten ihre Streuwirkung aufgrund eines Sprunges im Brechungsindex der beiden optischen Medien, Streuzentrum und umgebendes Gießharz. Günstig ist, wenn diese Streuzentren ausschließlich streuen d.h. selbst möglichst wenig absorbieren, also möglichst transparent sein sollten. Der Lichtverlust ist so trotz guter Streuwirkung gering. Will man bewußt den Lichtverlust dennoch erhöhen, kann man mit Titandioxid eine stärkere Streuung kombiniert mit stärkerer Absorption hervorrufen. Durch ausgewogenen Einsatz der beiden Streukomponenten läßt sich ein Streuungs-/Absorptions-Körper aufbauen, den man an einer geeigneten Stelle im Lichtweg plaziert. Mit ihm läßt sich ein weiter Bereich von zusätzlicher Streuung / Absorption überstreichen, mit dem sich eine sehr flexible Simulation der nicht-pulsatilen Gesamt-Streuung und GesamtAbsorption blutgefüllten Gewebes herstellen läßt.

Bringt man solche Streuungs/Absorptions-Körper in den geometrischen Lichtweg, so ersetzt er dort die Körperflüssigkeit (Blut) mit folgenden Konsequenzen:

- Die Streuung steigt oder fällt je nachdem, ob die Streuwirkung des Streuungs/Absorptions-Körpers größer oder kleiner ist als die verdrängte Körperflüssigkeit.
- Die Schichtdicke der Körperflüssigkeit verringert sich. Da aber vorteilhafterweise die Absorption des Streuungs/ Absorptions-Körpers deutlich geringer gewählt wird als die Absorption der Körperflüssigkeit, folgt daraus, daß
- die Körperflüssigkeits - spezifische Absorption fällt - die Lichtintensität steigt
- bei unveränderter pulsatiler Absorption steigt folglich die Modulationstiefe.
- Aus der Kombination von gestiegener Lichtintensität und erhöhter Modulationstiefe ergibt sich ein verbessertes Signal/Rausch-Verhältnis.

Es lassen sich somit die

- nicht-pulsatile absolute Absorption
- die nicht-pulsatile Streuung und
- die Modulationstiefe

auf gewünschte und/oder das Gewebe optimal simulierende Werte einstellen.

Obwohl die Lokalisation des Streuungs-/Absorptions-Körpers im Strahlengang prinzipiell unerheblich ist, gibt es bevorzugte Positionen: z.B. unmittelbar über den LEDs (Lichtsender) oder über dem Sensor Lichtempfänger. Ein Ort in der Mitte ist macht Schwierigkeiten hinsichtlich seiner Befestigung.

B) Absorption und Streuung und Blutanteil (Anteil der Körperflüssigkeit) im zeitlich **veränderlichen, pulsatilen Teil** Gewebe lassen sich wie folgt beeinflussen:

Die Streuung von Blut, hängt im wesentlichen vom Anteil an zellulären Bestandteilen ab, d.h. vom Hämatokrit. Die einfachste Beeinflussung ist folglich die Veränderung des Hämatokrit, die jedoch Streuung und Absorption gemeinsam beeinflußt. Wird es nötig, Streuung und Absorption getrennt zu modifizieren, kann man Streuung und Absorption durch Zusetzten von Partikeln im Sinne von Schwebekörpern erhöhen. Sie bilden winzige Streuzentren z.B.. kleine, einige μm große Kunststoffkugeln, ca. in der Größe von Erythrozyten. Sie entfalten ihre Streuwirkung aufgrund eines Sprunges im Brechungsindex der beiden optischen Medien: Streuzentrum und umgebendes Blut. Diese künstlichen Partikel können entweder streuen oder absorbieren oder beides. Um eine Entmischung zu vermeiden, sollte das spezifische Gewicht der Partikel auf das der Körperflüssigkeit abgestimmt sein. Liegt der Schwerpunkt auf einer Beeinflussung der Streuung, so wählt man vorteilhafterweise transparente, sphärische (kugelförmige) Partikel mit möglichst geringer Eigenabsorption. Will man bewußt die Absorption verändern, wählt man Partikel mit einer Absorption, ungefärbt (z.B. Titanoxid) oder gefärbt.

Diese Schwebekörper sollten in ihrem spezifischen Gewicht dem von Blut weitgehend entsprechen, damit keine Entmischung stattfindet sobald das Blut unbewegt ist. Man sollte ferner beachten, daß ein Zusatz von Streupartikeln zu Blut, dieses für CO-Oximeter-Analysen unbrauchbar macht, da die Ultraschall-"Hämolyse" dieser Partikel natürlich nicht gelingt.

Die Methoden der isolierten Beeinflussung von Streuung und Absorption der Körperflüssigkeit haben ihren Wert weniger in der Simulation der Körperflüssigkeit, die durch sich selbst ja bereits hinreichend simuliert ist. Vielmehr lassen sich durch isolierte Beeinflussung eines einzelnen Parameters komplexe Konstellationen leichter verstehen.

Optische Komponenten der Gewebs-Simulation:

| ABSORPTION GEWEBE konstant = nicht-pulsatil | STREUUNG GEWEBE konstant = nicht-pulsatil | ABSORPTION BLUT konstant = nicht-pulsatil |
|---|---|---|
| STREUUNG BLUT = KF konstant = nicht-pulsatil | ABSORPTION BLUT pulsatil | STREUUNG BLUT pulsatil |

Erfindungsgemäß wird der mittlere effektive Lichtweg zwischen Sender und Empfänger direkt mechanisch verändert unter der Maßgabe, daß die virtuelle optische Küvette dazwischen mit der zu untersuchenden Körperflüssigkeit, z.B. Blut angefüllt ist, zumindest in einem Umfang, der sicherstellt, daß zumindest der optische Raum, in dem die Änderung des mittleren effektiven Lichtwegs erfolgt, nahezu komplett von der Körperflüssigkeit (Blut) angefüllt ist, abgesehen z.B. von einem optischen Verdrängungskörper und/oder mechanischen Haltevorrichtungen und ähnlichem.

Meßtechnisch ist noch von besonderer Bedeutung, daß Sender und Empfänger immer dann nicht notwendigerweise einander zugewandt sein müssen, wenn die Körperflüssigkeit ein gewisses Maß an Streuung aufweist, wie das bei Blut der Fall ist. Die im Blut enthaltenen Zellen erzeugen eine Streuung, die, abhängig vom Hämoglobingehalt/ Hämatokrit, indirekte Lichtwege zuläßt. Sender und Empfänger können sogar vollständig voneinander orientiert sein. Bis zu Werten von 5g Hämoglobin/dl hinunter sind indirekte Lichtwege problemlos möglich. Da die Streuung im natürlichen Gewebe noch deutlich größer ist, ist sichergestellt, daß voneinander weg orientierte Sensor-Konstruktionsprinzipien nicht auf Schwierigkeiten stoßen.

In der Regel werden die Sensoren, insbesondere Sender und Empfänger, wenigstens teilweise von der Körperflüssigkeit umspült sein. Es ist jedoch auch möglich, sowohl Sender als auch Empfänger parallel oberhalb des Spiegels der Körperflüssigkeit anzuordnen, so daß praktisch eine Reflexionsmessung durchgeführt wird, wobei dann erfindungsgemäß Sender und/oder Empfänger vorzugsweise mit einer Frequenz, die der natürlichen Herzfrequenz des Menschen nahekommt, um ihre Ausgangslage in Schwingung versetzt werden, so daß das natürliche pulsatile Verhalten eines Gewebes, etwa eines menschlichen Fingers oder eines menschlichen Ohrläppchens meßtechnisch imitiert wird.

| zu simulierende optische Eigenschaft | Senkung der optischen Eigenschaft | Steigerung der optischen Eigenschaft |
|---|---|---|
| pulsatile Absorption | Senkung des Hb/Hk | Steigerung des Hb/Hk |
| pulsatile Streuung | nicht möglich (außer durch Senkung des Hb/Hk) | außer einer Steigerung des Hb/Hk: der Zusatz von streuenden Schwebeteilchen zum Blut (Schwebeteilchen sollten mit ihrem spezifischen Gewicht möglichst gut auf Blut abgestimmt sein) |
| nicht-pulsatile Absorption | Einfügen optischer Medien in den Lichtweg mit möglichst geringer Absorption, z.B. Glas oder Epoxyharz-Körper | unpraktikabel prinzipiell: Einfügen optischer Medien in den Lichtweg mit hoher Absorption, z.B. gefärbtes Glas |

(fortgesetzt)

| nicht-pulsatile Streuung | Einfügen optischer Medien in den Lichtweg mit im Vergleich zu Gewebe geringer Streuung, z.B. Milchglas oder Glaskugeln eingebettet in Epoxyharz-Körper (verschiedene Brechungs-Indizes Voraussetzung) | Einfügen optischer Medien in den Lichtweg mit im Vergleich zu Gewebe hoher Streuung, z.B. Milchglas oder Glaskugeln eingebettet in Epoxyharz-Körper (verschiedene Brechungs-Indizes Voraussetzung) |

Beschreibung der Zeichnungen:

Die Fig. 1 zeigt schematisch den Strahlengang in einer erfindungsgemäßen Kalibrationseinrichtung. **Blut** steht für Blut oder einen Blutersatz (im wesentlichen eine wäßrige Farbstofflösung). Um eine Lichtquelle herum (40) befindet sich die diffus streuende Farbstoffschicht. Die Pfeile zeigen den Strahlenverlauf (gestreute und nicht-gestreute Anteile) innerhalb der Meßvorrichtung an. Die auf die Fotodiode (**FD**) auftreffende Strahlung wird aufgezeichnet und ausgewertet. Es ergibt sich bei der erfindungsgemäßen Anordnung ein virtueller Küvettenrand (**VK**; Abstand von der Lichtquelle bei dem die rückgestreuten Anteile des Lichts zu schwach sind). Die diffus streuende Farbstoffschicht befindet sich im gesamten durch Streuung erfüllten optisch relevanten Raum.

Die Fig. 2 zeigt schematisch den erfindungsgemäßen Kalibrationsaufbau.

Die Figur 3 zeigt den prinzipiellen Aufbau einer Absorptionszelle insbesondere bei der Pulsoximetrie (theoretisch). Die Figur 4 gibt einen typischen idealisierten Verlauf der Absorption in einer solchen Zelle in Abhängigkeit von der Schichtdicke wieder.

Fig. 5 (nicht maßstabgetreu) veranschaulicht ein Ausführungsbeispiel eines Transmissions-Sensors (1) für die fetale Pulsoximetrie, wie in Patentschrift DE 3810008 C1 beschrieben. Er besteht aus einer spiralenförmigen Kanüle (2), die in einen Sensorkörper (3) eingebettet bzw. eingeklebt ist. In die Stahlspirale ist ein Fenster eingearbeitet, in die als Lichtquellen LEDs (4) transparent eingeklebt sind. Auf dem Boden des Sensorkörpers (3) ist eine Fotodiode (5) montiert, die nur durch eine dünne, optisch durchlässige Schicht getrennt, auf der Hautoberfläche zu liegen kommt, wenn die spiralförmige Kanüle (2) in die kindliche Kopfhaut eingeschraubt wird. Auf diese Weise befindet sich lebendes Gewebe zwischen den LEDs (4), die im Inneren des Gewebes zu liegen kommen und der Fotodiode (5), die auf der Kopfhaut zu liegen kommt. Sowohl die LEDs (4), als auch die Fotodiode (5) sind über das Sensorkabel (6), das z.B. als Kabel oder wie hier gezeichnet, als flexible Schaltung ausgeführt ist, angeschlossen. Das Sensorkabel (6) kann zudem EKG-Pole leiten. Die weiteren Bezugszeichen haben die folgende Bedeutung:

28 = Fenster
29 = Kontaktfläche

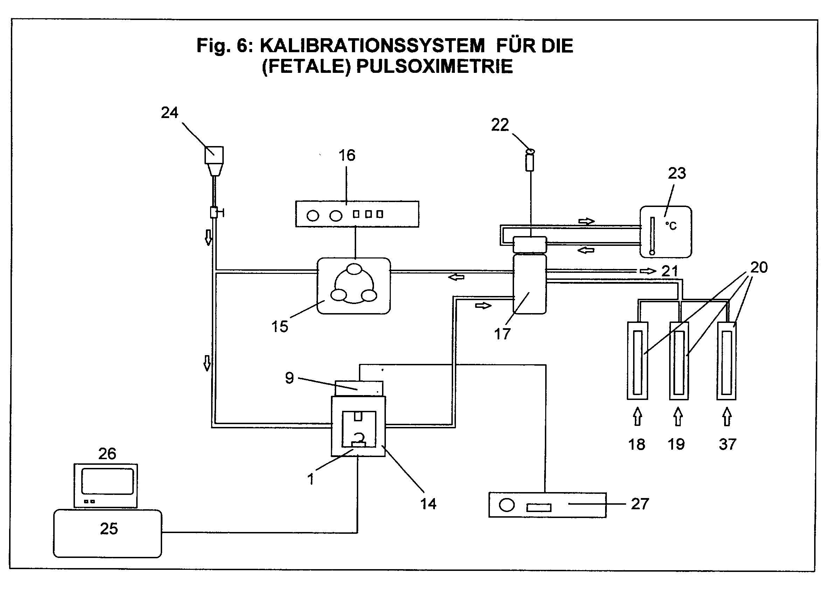

Fig. 6 (nicht maßstabgetreu) zeigt das Kalibrationssystem für die (fetale) Pulsoximetrie. Die Hauptkomponenten des Systems sind die Rollerpumpe (15), die als künstliches Herz Verwendung findet, der Faseroxigenator (17), der Blut mit einem Gasgemisch aus der Präzisionsflußregelung (20) äquilibriert, und die Plethysmographie-Simulations-Zelle (14), die den zu kalibrierenden Sensor (1) enthält. Die Präzisionsregelung (20) stellt mittels eines Flow-Einstellers für Stickstoff (18) und eines Flow-Einstellers für Sauerstoff (19) ein Stickstoff-Sauerstoff-Kohlendioxid-Gemisch so her, daß sich Sauerstoffkonzentrationen von ca. ½ - 5% ergeben. Dieses Gasgemisch fließt durch den Faseroxigenator (17) und strömt als Abluft (21) frei ab. Durch diesen Oxigenator (17) und durch die Plethysmographie-Simulations-Zelle (14) wird mit Hilfe der Rollerpumpe (15) mit Antrieb (16) (Schrittmotor) Blut in einen Kreislauf gepumpt. Dazu muß sichergestellt sein, daß das Blut für die Äquilibrierung im Faseroxigenator (17) eine definierte Temperatur aufweist. Dies wird mit Hilfe des temperatur-stabilisierten Wasserbades (23) bewerkstelligt, das Wasser konstanter Temperatur durch den Wärmetauscher des Faseroxigenators (17) pumpt. Die Rollerpumpe (15) wird von einer Antriebseinheit (16) angesteuert, damit der richtige Blutfluß im Kreislauf aus Rollerpumpe (16), Plethysmographie-Simulations-Zelle (14) und Faseroxigenator (17) erreicht wird. Da Blut zum Schäumen neigt, bedarf es einer Entlüftung (22), die gegebenenfalls kurzzeitig geöffnet wird. Die weiteren Bezugszeichen haben die folgende Bedeutung:

24 = Blutkonserve
25 = Pulsoximeter
26 = Anzeige
27 = Stromquelle für Magnetanregung

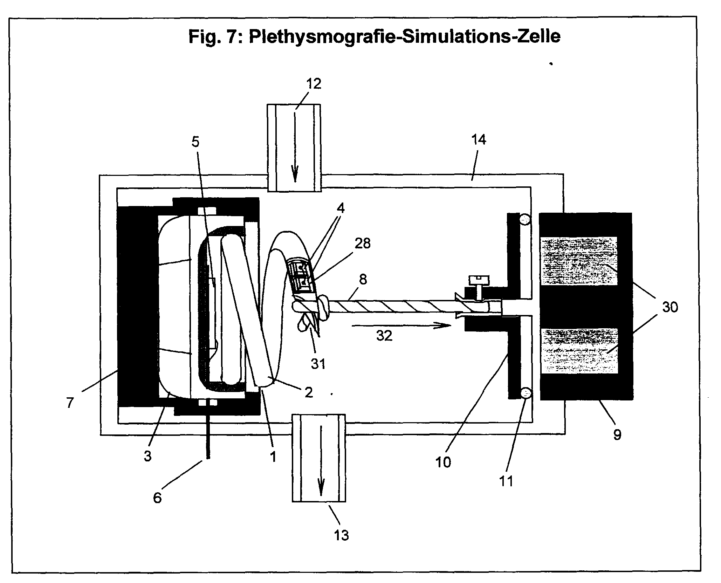

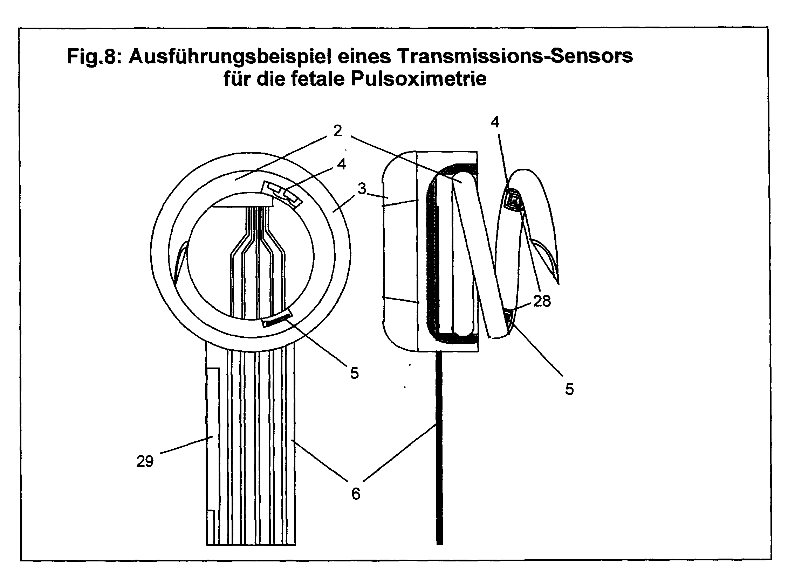

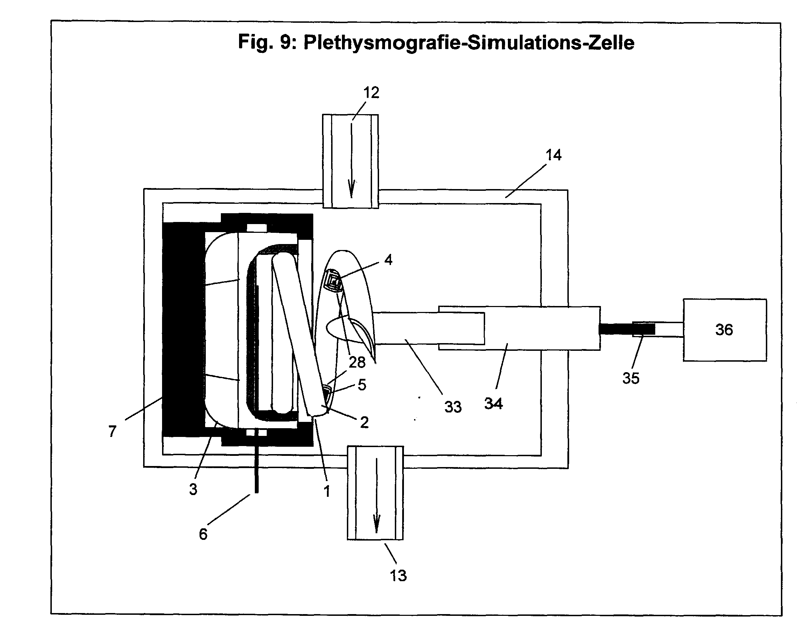

Fig. 7 (nicht maßstabgetreu) zeigt die Plethysmographie-Simulations-Zelle (14): Eine Sensorhalterung (7) fixiert den Sensor (1) fest und unbeweglich. Da die spiralenförmige Kanüle (2) die mechanischen Eigenschaften einer Feder aufweist, kann der mittlere effektive Lichtweg der LEDs (4) von der Fotodiode (5) durch Zug an der Nadelspitze variiert werden. Dieser Zug wird vom Elektromagneten (9) zunächst auf das Magnetjoch (10) ausgeübt und vom elastischen Ohrring (11) elastisch abgefedert. Ein nicht elastischer Zugfaden (8) überträgt die Bewegungen des Magnetjochs (10) auf die Nadelspitze. Schickt man repetitiv Strom durch den Elektromagneten (9), so wird auch die Spitze der spiralenförmigen Kanüle (2) rhythmisch bewegt. Da die ganze Plethysmographie-Simulations-Zelle (14) durch ihren Blutzufluß (12) und den Blutabfluß (13) von Blut durchströmt wird, befindet sich auch Blut zwischen der Lichtquelle (4) und dem Lichtempfänger (5). Es entsteht so eine repetitiv, z.B. periodisch veränderliche Schichtdicke an Blut, der zu fotometrierenden Körperflüssigkeit zwischen den LEDs (4) und der Fotodiode (5). Da durch den Zug mit dem inelastischen Zugfaden (8) nur Bewegungen in einer Richtung möglich sind, also nicht gedrückt werden kann, ist es vorteilhaft, die spiralförmige Kanüle vorzuspannen, z.B. indem man am Magnetjoch (10) über eine Gleichspannung (Offset) durch den Elektromagneten (9) konstant zieht. Die so vorgespannte Wegstrecke sollte größer sein als die halbe Amplitude der repetitiven Auslenkung.

Die weiteren Bezugszeichen haben die folgende Bedeutung:

3 = Sensorkörper
6 = Sensorkabel
28 = Fenster
30 = Magnetwicklung
31 = Nadel mit Zugfaden verklebt
32 = Zugrichtung

Es ist bei der praktischen Umsetzung der Erfindung zunehmend klar geworden, daß für die perfekte Gewebssimitation in der Kalibrationskammer in hohem Maß auch die Opazität zu beachten ist.. Die Diffusionswirkung von Blut ist erstaunlich gering - über lange Strecken bleibt im Blut offenbar eine Strahlrichtung erhalten. Der mit Abstand größte Beitrag zur Diffusionswirkung des Gewebes wird nicht vom Blut, sondern vom Gewebe selbst geleistet. Übersetzt man dies auf die Verhältnisse in der Kalibrationskammer, muß die Diffusität entweder vor dem LED-Fenster, oder vor dem Fotodioden-Fenster, oder durch den Modulator (Keil) erzeugt werden.

Es gibt ja prinzipiell zwei Möglichkeiten, optisch Diffusität zu erzeugen:

a) Man erzeugt durch Schleifen eine hohe Oberflächen-Unruhe und erzielt häufiges Brechen des Lichtes an den verschiedenen Facetten dieser Rauhigkeit. Dabei ist nur ein einzelner Brechungsindex im Spiel, der des Glases, wenn man den Umgebungs-Brechungsindex, z.B. von Luft oder von Blut/Wasser nicht berücksichtigt.

b) Man kann Diffusität auch durch multiple Übergänge zwischen verschiedenen Brechungsindizes erreichen. Dabei sind z.B. Glasperlen, die von einem Kunstharz umgeben sind, vorstellbar. Jede Glasperle wirkt hier als lichtablenkende Struktur, wobei die Zahl dieser Ablenkungszentren im Vergleich zu Schliffen wie unter a) aufgezeigt vergleichsweise gering ist. Entsprechend der geringen Zahl von Brech-Zentren ist der Lichtverlust solcher Anordnungen erstaunlich gering.

Beim Experimentieren mit UV-aushärtendem Epoxy-Kunstharzen, in die Glaskugeln eingeschlossen wurden, zeigte sich, daß silanisierte Glaskugeln nicht so günstig sind wie 3M-Glasbeads, das sind hohle, d.h. luftgefüllte Glaskugeln (wie transparente subminiatur Christbaumkugeln). Hier wird eine extrem hohe Diffusionswirkung dadurch erzeugt, daß ein Lichtstrahl beim Durchtreten durch eine Kugel multiple Brechungsindizes-Übergänge erfährt. Licht muß zunächst vom Kunststoff ins Glas, von Glas in Luft, und von Luft wieder in Glas, und von Glas wieder in Kunststoff übertreten. Es sind also 3 Brechungsindizes im Spiel (sieht man wieder von dem des Plasmas ab: der des Kunstharzes, der der Glaswand, und schließlich der der eingeschlossenen Luft.

Es wurden diffuse Glaskeile wie folgt hergestellt:

Duran-Vollglasstäbe mit 3 mm Durchmesser wurden an einem Stabende angeschliffen, indem man den Glaskeil in eine Handbohrmaschine einspannte und an die Spitze mit Daumen und Zeigefinger ein feuchtes 40 µm Schleifpapier andrückte. Das so aufgerauhte Glasende (besserer Halt des Epoxy-Harzes durch Aufrauhen beabsichtigt) wurde nun mit dem Glaskugel-gefüllten Epoxy-Harz (1/3 Glasbeads, 2/3 Epoxy-Harz [Prozentsatz in Volumenprozenten angegeben]) schichtweise umgeben, wobei darauf geachtet wurde, daß das Glaskugel-Epoxy-Harz-Gemisch das Glasende um ca. 5 mm verlängerte. Gleichzeitig wird versucht, das Epoxy-Harz nur unwesentlich über die Dicke des Glasstabes hinausragen zu lassen. Nach dem jeweiligen Auftragen des Epoxy-Harz-Glaskugel-Gemisches wird jeweils ausreichend und etwas länger mit der UV-Lichtleiterquelle ausgehärtet. Zum Schluß wird das Glasende mit einer Diamantscheibe angeschliffen, so daß sie leicht keilförmig mit einer planen Endfläche in die Sensorspirale eindringen kann.

Es wird durch das Anschleifen sichergestellt, daß auch die nun plane Spitze des Keils bereits die volle Diffusionswirkung zeigt, d.h., daß auch ganz vorne am Keil keinerlei Licht in Vorwärtsrichtung mehr auftritt.

Der so entstandene Glaskeil sieht zum Schluß aus wie ein gläsernes Streichholz mit weißem Kopf. Mit einem engen, nur minimal divergenten Helium-Neon-Laser wird geprüft, ob beim Aufstrahlen auf den Kopf des Keils noch ein Licht in Vorwärtsrichtung (z.B. an einer 1 m entfernten Wand) nachgewiesen werden kann. Ist der Strahl komplett unterbrochen, d.h. das Streichholz leuchtet heftig rot auf, der Laserstrahl an der Wand ist jedoch komplett verschwunden, nicht die winzigsten Moden sind noch zu sehen, dann ist der Keil als "gut gelungen" zu bewerten. Mit einem solchen Keil führten wir einen Kalibrationskammer-Versuch durch und bestimmten eine Kalibrationskurve für einen 660/940 nm Inside-Inside-Sensor und erhielten Omega-Werte von 0,3 - 3,95 für Sättigungen zwischen 100 und 0%, die ganz offensichtlich auf einer wenig gekrümmten Kurve (Hyperbel oder Parabel) lagen und die einzelnen Meßpunkte eine extrem geringe Standardabweichung aufwiesen.

Legende:

1 = Sensor
2 = spiralförmige Kanüle
3 = Sensorkörper
4 = LEDs
5 = Fotodiode
6 = Sensorkabel
7 = Sensorhalterung
8 = inelastischer Zugfaden
9 = Elektromagnet
10 = Magnetjoch
11 = elastischer O-Ring
12 = Blutzufluß
13 = Blutabfluß
14 = Kalibrationszelle (künstliches Gewebe)
15 = Rollerpumpe (künstliches Herz)
16 = Antrieb für Rollerpumpe (Schrittmotor)
17 = Faseroxigenator (künstliche Lunge)
18 = Stickstoff-Zufluß
19 = Sauerstoff-Zufluß
20 = Präzisions-Flußregelung
21 = Abluft
22 = Entlüftung
23 = Temperaturstabiles Wasserbad
24 = Blutkonserve
25 = BLM-Pulsoximeter
26 = Anzeige
27 = Stromquelle für Magnetanregung
28 = Fenster
29 = Kontaktfläche
30 = Magnetwicklung
31 = Nadel mit Zugfaden verklebt
32 = Zugrichtung
33 = Diffuser Glaskeil
34 = Keilhalterung
35 = Kupplung
36 = Motor
37 = $CO_2$-Zufluß
38 = herausgestreuter Strahl
39 = Licht, zu schwach bezogen auf das Gesamtsignalniveau
40 = Lichtquelle
41 = Blut bzw. in Flüssigkeit gelöster Farbstoff mit der Konzentration c, streuendes Medium Voraussetzung
42 = Fotodiode
43 = Physikalischer Küvettenrand
44 = virtueller Küvettenrand

45 =   gestreuter Strahl

**Patentansprüche**

1. Verfahren zur Validierung von Vorrichtungen zur Fotometrie lebender Gewebe, insbesondere von Pulsoximetern, welches die folgenden Schritte umfaßt:

   - In vitro-Einstellen einer bestimmten Konzentration einer in einer Körperflüssigkeit zu erfassenden Substanz,
   - Transportieren der auf die bestimmte Konzentration eingestellten Körperflüssigkeit zu wenigstens einer Meßzelle,
   - Durchstrahlen der Körperflüssigkeit mittels wenigstens einem Lichtsender,
   - Erfassen der Lichtintensität in wenigstens einem spektralen Fenster mittels wenigstens einem Lichtempfänger zur Bestimmung mindestens eines geeigneten Parameters in der Körperflüssigkeit, welche in der Meßzelle in einem Behälter aufgenommen ist,
   - Verändern der effektiven Lichtweglänge durch die Körperflüssigkeit aktiv auf definierte Art, ohne die Körperflüssigkeit zur Übertragung von Kräften einzusetzen,
   - Herstellen wenigstens einer Korelation zwischen der Konzentration der Substanz einerseits und dem geeigneten Parameter andererseits, wobei die Körperflüssigkeit den optischen Raum zwischen Sender und Empfänger so einnimmt, daß die Intensität der Summe der vom Sender zum Empfänger durch die Körperflüssigkeit zurückgelegten effektiven Lichtweglängen nicht durch den Behälter der Körperflüssigkeit beschränkt ist.

2. Verfahren nach Anspruch 1, dadurch gekennzeichnet, daß die Validierung eine Kalibration, insbesondere eine in vitro-Kalibration umfaßt.

3. Verfahren nach einem der Ansprüche 1 oder 2, dadurch gekennzeichnet, daß als Vorrichtung zur Fotometrie lebender Gewebe ein Pulsoximeter validiert, insbesondere kalibriert, wird.

4. Verfahren nach einem der Ansprüche 1 bis 3, dadurch gekennzeichnet, daß als Körperflüssigkeit Blut, insbesondere menschliches, vorzugsweise fetales Blut, verwendet wird.

5. Verfahren nach einem der Ansprüche 1.bis 4, dadurch gekennzeichnet, daß als Konzentration einer in einer Körperflüssigkeit zu erfassenden Substanz die Sauerstoff-Sättigung verwendet wird.

6. Verfahren nach einem der Ansprüche 1 bis 5, dadurch gekennzeichnet, daß man Licht zweier spektraler Fenster, bevorzugt Rot- und Infrarotlicht, insbesondere für die Pulsoximetrie verwendet, dessen Intensität nach Durchdringen der Körperflüssigkeit mittels eines Fotosensors erfaßt wird.

7. Verfahren nach einem der Ansprüche 1 bis 6, dadurch gekennzeichnet, daß die Änderung der effektiven Absorptionslänge durch die Änderung der Blutschichtdicke als Folge einer Änderung des mittleren effektiven Lichtwegs zwischen Sender und Empfänger mittels magnetischer und/oder mechanischer Kräfte insbesondere repetitiv, vorzugsweise periodisch bewirkt wird.

8. Verfahren nach einem der Ansprüche 1 bis 6, dadurch gekennzeichnet, daß die Änderung der effektiven Absorptionslänge durch die Änderung der Blutschichtdicke als Folge einer Verdrängung von Blut durch Einbringen eines Verdrängungskörpers zwischen Sender und Empfänger eine Änderung des mittleren effektiven Lichtweges zwischen Sender und Empfänger mittels magnetischer und/oder mechanischer Kräfte insbesondere repetitiv, vorzugsweise periodisch bewirkt wird, wobei die eigene Absorption des Verdrängungskörpers entweder bekannt oder vorzugsweise besonders gering ist.

9. Verfahren nach Anspruch 8, dadurch gekennzeichnet, daß man als Verdrängungskörper einen, bezogen auf das spektrale Fenster optisch durchlässigen Körper, beispielsweise aus Glas, mit variabler Dicke verwendet, vorzugsweise einen Glaskeil, wobei der Verdrängungskörper beispielsweise licht-streuende Eigenschaften aufweist vorzugsweise so ausgeprägt, daß bei Durchstrahlung des Verdrängungskörpers mit extrem parallelem Licht (Laser) in Richtung des Lichtstrahls keine erhöhte Lichtintensität nachweisbar ist.

10. Verfahren nach Anspruch 8 oder 9, dadurch gekennzeichnet, daß man als Verdrängungskörper einen optisch stark streuenden durchlässigen Körper, beispielsweise aus Glas, mit variabler Dicke verwendet, vorzugsweise einen diffusen Glaskeil.

11. Verfahren nach Anspruch 10, dadurch gekennzeichnet, daß man als Körper eine rotierende, optisch durchlässige Scheibe mit veränderlicher Dicke verwendet.

12. Verfahren nach einem der Ansprüche 1 bis 11, dadurch gekennzeichnet, daß der Sender und/oder Empfänger zur Verringerung der nicht-pulsatilen Absorptionslänge der Körperflüssigkeit, insbesondere Blut, optisch durchlässig beschichtet wird, wobei man als bevorzugte Beschichtung ein Glas oder einen Kunststoff, insbesondere Kunstharz, vorzugsweise Epoxyharz verwendet.

13. Verfahren nach einem der Ansprüche 1 bis 12, dadurch gekennzeichnet, daß die effektive Absorptionslänge zur Vergrößerung der nicht-pulsatilen Lichtintensität durch Einbringen einer im Vergleich zur Körperflüssigkeit besser lichtdurchlässigen Schicht verringert wird, wobei man als bevorzugte Beschichtung ein transparentes Glas oder Kunstharz oder in Kunstharz dispergierte Glasperlen von vorzugsweise 1 um mittleren Durchmessers verwendet.

14. Verfahren nach einem der Ansprüche 1 bis 13, dadurch gekennzeichnet, daß man Teilchen in die Schicht auf dem Sender und/oder Empfänger eindispergiert, wobei Glasperlen, insbesondere solche von etwa 1 μm Durchmesser, oder Titandioxid bevorzugt sind.

15. Verfahren nach einem der Ansprüche 1 bis 14, dadurch gekennzeichnet, daß man in die Körperflüssigkeit, insbesondere Blut, zur besseren Gewebeimitation Streuteilchen einsuspendiert, insbesondere solche aus Kunststoff wie PE, HDPE oder dergleichen, welche in etwa die spezifische Dichte der Körperflüssigkeit aufweisen, und welche einen solchen Durchmesser aufweisen, daß sie einen Faseroxigenator passieren können.

16. Verfahren nach einem der Ansprüche 1 bis 15, dadurch gekennzeichnet, daß man als geeigneten Parameter die Variable $\Omega$ verwendet, welche gemäß folgender besonders genauer Gleichung definiert ist:

$$\Omega = \frac{\ln\left(\dfrac{I_{out\,max\,\lambda 1}}{I_{out\,min\,\lambda 1}}\right)}{\ln\left(\dfrac{I_{out\,max\,\lambda 2}}{I_{out\,min\,\lambda 2}}\right)}$$

17. Verfahren nach einem der Ansprüche 1 bis 16, dadurch gekennzeichnet, daß man als Zusammenhang insbesondere eine Beziehung zwischen $\Omega$ einerseits und einer der möglichen Sauerstoff-Sättigungs-Definitionen andererseits wertet, insbesondere der funktionellen Sauerstoff-Sättigung [$SaO_{2func}$] gemäß folgender Gleichung:

$$SaO_{2(func)} = \frac{Hb_{oxi}}{Hb_{oxi} + Hb_{deoxi}}$$

und folgende Gleichung zur Kalibration gegebenenfalls unter Erstellung einer Kalibrationskurve verwendet wird:

$$SaO_{2(func)} = f(\Omega)$$

18. Verfahren nach einem der vorstehenden Ansprüche,
dadurch gekennzeichnet, daß

die Körperflüssigkeit Blut ist und die Substanz der Sauerstoff der momentan gemischten, venösen, kapillären und arteriellen Sauerstoff-Sättigung des Blutes einer vollständig von der Durchblutung abgebundenen Extremität sowohl quasi-pulsoximetrisch während der langsamen Desaturierung des gesamten Kapillarbettes der Extremität ist,
das Verändern der effektiven Lichtweglänge mittels künstlicher Blutfüllungsschwankungen des transmittierten Gewebes durch von außen in hinreichender Nähe der Vorrichtung einwirkende Druckschwankungen bewirkt wird sowie,
daß durch ein geeignetes Referenzverfahren die wirkliche momentane Gewebe-Sauerstoff-Sättigung bestimmt wird.

**19.** Verfahren nach einem der vorstehenden Ansprüche,
dadurch gekennzeichnet, daß

- als Lichtsender zwei Sensor-LED's dienen, und als Lichtempfänger eine Sensor-Fotodiode,
- ein passender Trenneinschub dazwischen so eingebracht wird, daß er das Licht der Sensor-LED's ausreichend von der Sensor-Fotodiode abschirmt,
- der Trenneinschub mindestens zwei weitere Fotodioden trägt, die gegenüber den Sensor-LEDs angeordnet sind, von denen eine der zwei weiteren Fotodioden einen optischen Filter trägt, so daß sich durch eine geeignete Elektronik die beiden Sensor-LED-Signale analysieren lassen und vollständig analog zur Verfügung stehen, und
- der Trenneinschub mindestens eine weitere LED trägt, die die elektronisch modulierten Sensor-LED-Signale in Lichtsignale umwandelt und in die Sensor-Fotodiode derart einspeist, daß sich für die Vorrichtung, insbes. den Pulsoximeter, ein zeitlich veränderliches Signal ergibt, das identisch mit einem herkömmlichen Pulsoximetriesignal ist, das dadurch entsteht, daß sich lebendes Gewebe im Sensor befindet.

**20.** Verfahren nach Anspruch 19,
dadurch gekennzeichnet, daß

- durch die Modulation der rekonstruierten Sensor-LED-Signale mittels der von einem Mikroprozessor generierten Gewebsfunktion ein vollständiges, valides Pulsoximetriesignal entsteht,
- das über die LED des Trenneinschubes in die Sensor-Fotodiode der Vorrichtung, insbes. des Pulsoximeters, eingespeist wird und zu einer Auswertung benutzt wird, die sowohl die Sauerstoff-Sättigung des Blutes als auch die Herzfrequenz ergibt, und
- daß durch Modulation mit einem geeignet generierten Signal sich Eigenschaften des Gewebes als auch der Vorrichtung simulieren lassen.

**21.** Vorrichtung zur Durchführung des Verfahrens nach einem der vorstehenden Ansprüche mit:

- wenigstens einer Einrichtung zum Einstellen der Konzentration einer in einer Körperflüssigkeit zu erfassenden Substanz;
- wenigstens einer Transporteinrichtung, womit die auf eine bestimmte Konzentration eingestellte Körperflüssigkeit zu wenigstens einer Meßzelle gebracht wird;
- wenigstens einem Sender und wenigstens einem Empfänger, welche in der Meßzelle angeordnet sind, so daß der mittlere effektive Lichtweg zueinander dynamisch auf definierte Art veränderbar ist, ohne daß zur Änderung des mittleren effektiven Lichtwegs die Körperflüssigkeit zur Übertragung von Kräften auf Sender und/oder Empfänger eingesetzt . wird; und
- einer Einrichtung, welche eine mittels des Empfängers erfaßte Lichtintensität wenigstens eines spektralen Fensters, welche die Körperflüssigkeit durchdrungen hat, in Form wenigstens eines geeigneten Parameters ausgibt und in einen Zusammenhang zwischen der Konzentration einerseits und dem Parameter andererseits bringt.

**22.** Vorrichtung nach Anspruch 21,
wobei die Veränderung des mittleren effektiven Lichtweges durch Anordnung einer optischen Diskontinuität mit veränderbarer Dicke im optischen Raum zwischen Sender und Empfänger und Veränderung dessen Dicke vorgesehen ist.

**Claims**

**1.** A method of validating devices for the photometry of living tissue, in particular pulse oximeters, which includes the following steps:

- in vitro adjusting a given concentration of a substance to be detected in a body fluid,
- transporting the body fluid which is adjusted to the given concentration to at least one measuring cell,
- transilluminating the body fluid by means of at least one light transmitter,
- detecting the light intensity in at least spectral window by means of at least one light receiver for determining at least one suitable parameter in the body fluid which is contained in the measuring cell in a container,

- varying the effective light path length through the body fluid actively in a defined manner without using the body fluid for the transmission of forces, and

- producing at least one correlation between the concentration of the substance on the one hand and the suitable parameter on the other hand, wherein the body fluid so occupies the optical space between the transmitter and the receiver that the intensity of the sum of the effective light path lengths covered from the transmitter to the receiver through the body fluid is not limited by the container of the body fluid.

2. A method according to claim 1 characterised in that validation includes a calibration operation, in particular in vitro calibration.

3. A method according to one of claims 1 and 2 characterised in that a pulse oximeter is validated, in particular calibrated, as the device for the photometry of living tissue.

4. A method according to one of claims 1 to 3 characterised in that blood, in particular human blood, preferably foetal blood, is used as the body fluid.

5. A method according to one of claims 1 to 4 characterised in that oxygen saturation is used as the concentration of a substance to be detected in a body fluid.

6. A method according to one of claims 1 to 5 characterised in that light of two spectral windows, preferably red and infra-red light, is used, in particular for pulse oximetry, the intensity thereof being determined by means of a photosensor after passing through the body fluid.

7. A method according to one of claims 1 to 6 characterised in that the variation in the effective absorption length is in particular repetitively and preferably periodically caused by the variation in the thickness of the layer of blood as a consequence of a variation in the mean effective light path between the transmitter and receiver by means of magnetic and/or mechanical forces.

8. A method according to one of claims 1 to 6 characterised in that the variation in the effective absorption length is caused in particular repetitively and preferably periodically by the variation in the thickness of the layer of blood as a consequence of a displacement of blood by the introduction of a displacement body between the transmitter and the receiver causing a variation in the mean effective light path between the transmitter and the receiver by means of magnetic and/or mechanical forces, wherein the absorption of the displacement body itself is either known or is preferably particularly small.

9. A method according to claim 8 characterised in that the displacement body used is a body which is optically transmissive in relation to the spectral window, for example of glass, of variable thickness, preferably a glass wedge, wherein the displacement body has for example light-scattering properties preferably so pronouncedly that no increased light intensity is detectable upon transillumination of the displacement body with extremely parallel light (laser) in the direction of the light beam.

10. A method according to claim 8 or claim 9 characterised in that the displacement body used is an optically strongly scattering transmissive body, for example of glass, of variable thickness, preferably a diffuse glass wedge.

11. A method according to claim 10 characterised in that the body used is a rotating, optically transmissive disc of variable thickness.

12. A method according to one of claims 1 to 11 characterised in that the transmitter and/or receiver is optically transmissively coated for reducing the non-pulsatile absorption length of the body fluid, in particular blood, wherein the preferred coating is a glass or a plastics material, in particular synthetic resin, preferably epoxy resin.

13. A method according to one of claims 1 to 12 characterised in that to increase the non-pulsatile light intensity the effective absorption length is reduced by the introduction of a layer which is better light-transmissive in comparison with the body fluid, wherein the preferred coating used is a transparent glass or synthetic resin or glass beads dispersed in synthetic resin and of a mean diameter which is preferably 1 $\mu$m.

14. A method according to one of claims 1 to 13 characterised in that particles are dispersed into the layer on the transmitter and/or receiver, wherein glass beads, in particular those of about 1 μm in diameter, or titanium oxide, are preferred.

15. A method according to one of claims 1 to 14 characterised in that for better tissue imitation scattering particles are suspended in the body fluid. in particular blood, in particular particles comprising plastics material such as PE, HDPE or the like, which are of approximately the specific density of the body fluid and which are of such a diameter that they can pass through a fibre oxygenator.

16. A method according to one of claims 1 to 15 characterised in that the suitable parameter used is the variable $\Omega$ which is defined in accordance with the following particularly accurate equation:

$$\Omega = \frac{\ln\left(\dfrac{I_{out\ max\ \lambda1}}{I_{out\ min\ \lambda1}}\right)}{\ln\left(\dfrac{\cdot I_{out\ max\ \lambda2}}{I_{out\ min\ \lambda2}}\right)}$$

17. A method according to one of claims 1 to 16 characterised in that the relationship evaluated is in particular a relationship between $\Omega$ on the one hand and one of the possible oxygen saturation definitions on the other hand, in particular the functional oxygen saturation [$SaO_{2func}$] in accordance with the following equation:

$$SaO_{2(func)} = \frac{Hb_{oxi}}{Hb_{oxi} + Hb_{deoxi}}$$

and the following equation is used for calibration, possibly with the production of a calibration curve:

$$SaO_{2(func)} = f(\Omega)$$

18. A method according to one of the preceding claims characterised in that

the body fluid is blood and the substance is the oxygen of the presently mixed venous, capillary and arterial oxygen saturation of the blood of an extremity which is completely cut off from the circulation both quasi-pulse oximetrically during the slow desaturation of the total capillary bed of the extremity,
the variation in the effective light path length is produced by means of artificial blood filling fluctuations of the transmitted tissue by pressure fluctuations acting from the the exterior in sufficient proximity of the apparatus, and
the actual present tissue oxygen saturation is determined by a suitable reference method.

19. A method according to one of the preceding claims characterised in that

- two sensor-LEDs serve as the light transmitter and a sensor photodiode serves as the light receiver,
- a suitable separating insert is so introduced therebetween that it sufficiently shields the light of the sensor-LEDs from the sensor photodiode.
- the separating insert carries at least two further photodiodes which are arranged opposite to the sensor-LEDs, of which one of the two further photodiodes carries an optical filter so that by suitable electronics the two sensor-LED signals can be analysed and are fully available in analog form, and
- the separating insert carries at least one further LED which converts the electronically modulated sensor-LED signals into light signals and so feeds them into the sensor photodiode that for the apparatus, in particular the pulse oximeter, there is a signal which is variable in respect of time and which is identical to a conventional pulse oximetry signal which is produced by living tissue being disposed in the sensor.

20. A method according to claim 19 characterised in that

- a complete valid pulse oximetry signal is produced by modulation of the reconstructed sensor-LED signals by means of the tissue function generated by a microprocessor,
- which signal is fed by way of the LED of the separating insert into the sensor photodiode of the apparatus, in particular the pulse oximeter. and is used for an evaluation operation which gives both the oxygen saturation of the blood and also the heart rate, and
- properties of the tissue and also the apparatus can be simulated by modulation with a suitably generated signal.

21. Apparatus for carrying out the method according to one of the preceding claims comprising:

- at least one device for adjusting the concentration of a substance to be detected in a body fluid;
- at least one transport device with which the body fluid which has been adjusted to a given concentration is brought to at least one measuring cell;
- at least one transmitter and at least one receiver which are arranged in the measuring cell so that the mean effective light path is variable relative to each other dynamically in a defined manner, without the body fluid being used for the transmission of forces to transmitter and/or receiver for varying the mean effective light path; and
- a device which outputs a light intensity detected by means of the receiver of at least once spectral window which has passed through the body fluid in the form of at least one suitable parameter and brings it into a relationship between the concentration on the one hand and the parameter on the other hand.

22. Apparatus according to claim 21

wherein the variation in the mean effective light path is provided by the arrangement of an optical discontinuuity of variable thickness in the optical space between the transmitter and the receiver and a variation in the thickness thereof.

**Revendications**

1. Procédé pour la validation de dispositifs de photométrie de tissus vivants en particulier de pulsoximètres, procédé qui comprend les étapes suivantes :

- le réglage en éprouvette d'une certaine concentration d'une substance à relever dans un liquide corporel,

- le transport du liquide corporel réglé à la concentration déterminée à au moins une cellule de mesure,

- l'exposition au rayonnement du liquide corporel au moyen d'au moins un émetteur lumineux,

- la saisie de l'intensité lumineuse dans au moins une fenêtre spectrale au moyen d'un récepteur de lumière pour la détermination d'au moins un paramètre approprié dans le liquide corporel qui doit être réceptionné dans la cellule de mesure dans un récipient,

- la modification de la longueur effective du trajet lumineux à travers le liquide corporel, actif de manière définie sans employer le liquide corporel pour le transfert de forces,

- l'établissement d'au moins une corrélation entre la concentration de la substance d'une part et le paramètre approprié d'autre part, le liquide corporel occupant l'espace optique entre l'émetteur et le récepteur de sorte que l'intensité de la somme des longueurs de parcours lumineux effectifs parcourus par le liquide corporel de l'émetteur au récepteur ne soit pas limitée par le récipient du liquide corporel.

2. Procédé selon la revendication 1, caractérisé en ce que la validation comprend un calibrage, en particulier un calibrage en éprouvette.

3. Procédé selon l'une des revendications 1 ou 2, caractérisé en ce qu'on valide en particulier calibre à l'aide d'un pulsoximètre en tant que dispositif de photométrie de tissus vivants.

4. Procédé selon l'une des revendications 1 à 3, caractérisé en ce qu'on utilise en tant que liquide corporel du sang en particulier du sang humain, de préférence foetal.

**5.** Procédé selon l'une des revendications 1 à 4, caractérisé en ce qu'on utilise en tant que concentration d'une substance à saisir dans un liquide corporel la saturation en oxygène.

**6.** Procédé selon l'une des revendications 1 à 5, caractérisé en ce qu'on utilise la lumière de deux fenêtres spectrales de préférence la lumière rouge et la lumière infrarouge, en particulier pour la pulsoximétrie dont l'intensité est mesurée au moyen d'un capteur photo après la traversée du liquide corporel.

**7.** Procédé selon l'une des revendications 1 à 6, caractérisé en ce que la modification de la longueur effective d'absorption est provoquée par la variation de l'épaisseur de la couche de sang à la suite d'une modification du parcours lumineux moyen effectif entre l'émetteur et le récepteur au moyen de forces magnétiques et/ou mécaniques en particulier de manière répétitive, de préférence périodique.

**8.** Procédé selon l'une des revendications 1 à 6, caractérisé en ce que la modification de la longueur effective d'absorption est provoquée par la variation de l'épaisseur de la couche de sang à la suite d'un refoulement du sang par incorporation d'un corps de refoulement entre l'émetteur et le récepteur et par une modification du parcours lumineux moyen entre l'émetteur et le récepteur au moyen de forces magnétiques et/ou mécaniques en particulier de manière répétitive, de préférence périodique, la propre absorption du corps de refoulement étant soit connue ou soit de préférence faible.

**9.** Procédé selon la revendication 8, caractérisé en ce qu'on utilise en tant que corps de refoulement, un corps optiquement transparent par rapport à la fenêtre spectrale, par exemple en verre d'épaisseur variable, de préférence un cône en verre, le corps de refoulement présentant par exemple des propriétés de diffusion de la lumière, et étant de préférence marqué de sorte que quand la lumière extrêmement parallèle (laser) traverse le corps de refoulement, on ne peut constater aucune intensité lumineuse plus élevée dans le sens du rayon lumineux.

**10.** Procédé selon la revendication 8 ou 9, caractérisé en ce qu'on utilise en tant que corps de refoulement un corps transparent à forte diffusion optique, par exemple en verre d'épaisseur variable, par exemple un cône en verre diffus

**11.** Procédé selon la revendication 10, caractérisé en ce qu'on utilise en tant que corps un disque rotatif, transparent d'épaisseur variable.

**12.** Procédé selon l'une des revendications 1 à 11, caractérisé en ce que l'émetteur et/ou le récepteur est recouvert d'une couche optique transparente pour la diminution de la longueur d'absorption non pulsative du liquide corporel, en particulier du sang, auquel cas on utilise en tant que revêtement préféré un verre ou une matière plastique en particulier de la résine synthétique de préférence de la résine Epoxy.

**13.** Procédé selon l'une des revendications 1 à 12, caractérisé en ce que pour augmenter l'intensité lumineuse non pulsative, la longueur d'absorption effective est diminuée par incorporation d'une couche laissant mieux passer la lumière en comparaison d'un liquide corporel, de sorte qu'on utilise en tant que revêtement préféré un verre transparent ou une résine synthétique ou des perles de verre dispersées dans la résine synthétique d'un diamètre moyen de 1 μm de préférence.

**14.** Procédé selon l'une des revendications 1 à 13, caractérisé en ce qu'on envoie dans la couche des particules sur l'émetteur et/ou le récepteur, en accordant la préférence au dioxyde de titane ou à des perles de verre, en particulier celles d'un diamètre moyen de 1 pm de préférence.

**15.** Procédé selon l'une des revendications 1 à 14, caractérisé en ce qu'on met en suspension dans le liquide corporel, en particulier du sang, pour une meilleure imitation du tissu, des particules de dispersion, en particulier celles en matière synthétique comme le PE, HDPE ou similaires qui présentent approximativement la densité spécifique du liquide corporel et qui présentent un diamètre permettant de passer un oxigénateur à fibre.

**16.** Procédé selon l'une des revendications 1 à 14, caractérisé en ce qu'on utilise en tant que paramètre approprié la variable $\Omega$ qui est définie par l'équation suivante particulièrement exacte :

$$\Omega = \frac{\ln\left(\dfrac{I_{out\ max\ \lambda 1}}{I_{out\ min\ \lambda 1}}\right)}{\ln\left(\dfrac{I_{out\ max\ \lambda 2}}{I_{out\ min\ \lambda 2}}\right)}$$

17. Procédé selon l'une des revendications 1 à 16, caractérisé en ce qu'on analyse une corrélation en particulier une relation, d'une part, entre $\Omega$ et, d'autre part, l'une des définitions possibles de saturation en oxygène en particulier la saturation fonctionnelle en oxygène [$SaO_{2func}$] suivant l'équation suivante :

$$SaO_{2func} = \frac{Hb_{oxi}}{Hb_{oxi} + Hb_{deoxi}}$$

et on utilise l'équation suivante pour le calibrage le cas échéant en réalisation une courbe de calibrage,

$$SaO_{2func} = f\left(\Omega\right)$$

18. Procédé selon l'une des revendications précédentes, caractérisé en ce que le liquide corporel est du sang et la substance est constituée par l'oxygène de la saturation artérielle, capillaire, veineuse et momentanément mélangée du sang en oxygène d'une extrémité complètement liée par la circulation sanguine est quasi pulsoximétrique pendant la lente désaturation de tout le lit capillaire de l'extrémité, en ce que la modification de la longueur effective du parcours lumineux est provoquée au moyen de variations artificielles de la charge sanguine du tissu transmission par des variations de pression agissant de l'extérieur à proximité suffisante du dispositif, et en ce que la saturation réelle momentanée du tissu en oxygène est définie par un procédé de référence approprié.

19. Procédé selon l'une des revendications précédentes, caractérisé en ce que

- pour l'émetteur de lumière, on se sert de deux capteurs DEL et pour le récepteur de lumière, un capteur photodiode,

- un dispositif insérable de séparation approprié est intercalé de manière à faire écran de manière suffisante pour la lumière des capteurs DEL vis-à-vis du capteur photodiode,

- le dispositif de séparation porte au moins deux autres photodiodes qui sont disposées en regard des capteurs DEL, dont l'une des deux autres photodiodes porte un filtre optique de sorte qu'une électronique appropriée est en mesure d'analyser les deux signaux du capteur DEL qui sont entièrement disponibles de manière analogique et

- le dispositif de séparation porte au moins une autre DEL qui convertit les signaux du capteur DEL modulés électroniquement en signaux lumineux et les envoie au capteur photodiode de sorte qu'il en résulte pour le dispositif en particulier le pulsoximètre un signal à temps modifié qui est identique à un signal traditionnel en pulsoximétrie se manifestant quand un tissu vivant se trouve dans le capteur.

20. Procédé selon la revendication 19, caractérisé en ce que

- du fait de la modulation des signaux reconstitués du capteur DEL, on obtient au moyen de la fonction tissulaire générée par un microprocesseur un signal complet valide de pulsoximétrie,

- qui est envoyé par l'intermédiaire de la DEL du dispositif insérable de séparation au capteur photodiode du dispositif en particulier du pulsoximètre et est utilisé en vue d'une évaluation qui donne aussi bien la saturation en oxygène du sang qu'également le rythme cardiaque et

- par la modulation à l'aide d'un signal généré de manière appropriée, on peut simuler des propriétés du tissu et du dispositif.

21. Dispositif pour la mise en oeuvre du procédé selon l'une des revendications précédentes, comprenant

- au moins un dispositif pour le réglage de la concentration d'une substance à mesurer dans un liquide corporel,

- au moins un dispositif de transport avec lequel le liquide corporel réglé à une certaine concentration est amené à au moins une cellule de mesure ,

- au moins un émetteur et au moins un récepteur qui sont disposés dans la cellule de mesure de sorte que la dynamique relative du parcours lumineux moyen effectif peut être modifiable de manière définie sans que pour la modification du parcours lumineux effectif moyen, le liquide corporel soit utilisé pour la transmission de forces entre l'émetteur et/ou le récepteur ; et

- un dispositif qui délivre sous forme d'au moins un paramètre approprié une intensité lumineuse saisie au moyen du récepteur d'au moins une fenêtre spectrale, cette intensité ayant traversé le liquide corporel, et apporte une corrélation entre la concentration d'une part et le paramètre d'autre part.

22. Dispositif selon la revendication 21, où la modification du parcours lumineux effectif moyen est prévue par la disposition d'une discontinuité optique d'épaisseur variable dans l'espace optique entre l'émetteur et le récepteur, la modification de cette épaisseur étant également prévue.

**Fig. 1:**

**Fig. 2:**

PUMPE =
HERZ

PLETHYSMOGRAFIE -
SIMULATIONS -
ZELLE (PSC)
=
KÜNSTLICHES GEWEBE

(FASER-) OXIGENATOR
=
LUNGE / PLAZENTA
mit Wärmetauscher zur
Einstellung der Bluttemperatur

**Fig. 3**

$I_0 \longrightarrow$      $I_{out}$

Schichtdicke

0     d   d+δ

**Fig. 4**

Lichtintensität

Io

Ioutmax

Ioutmin

Schichtdicke

d   ε+d

# Fig.5: Ausführungsbeispiel eines Transmissions-Sensors für die fetale Pulsoximetrie

Fig. 6: KALIBRATIONSSYSTEM FÜR DIE (FETALE) PULSOXIMETRIE

EP 0 777 854 B1

Fig. 7: Plethysmografie-Simulations-Zelle

**Fig.8: Ausführungsbeispiel eines Transmissions-Sensors für die fetale Pulsoximetrie**

EP 0 777 854 B1

Fig. 9: Plethysmografie-Simulations-Zelle